# EUROPÄISCHE PATENTSCHRIFT

(11) **EP 2 170 245 B1**
(45) Veröffentlichungstag und Bekanntmachung des Hinweises auf die Patenterteilung: **28.11.2012**
(21) Anmeldenummer: 08760669.5
(22) Anmeldetag: 06.06.2008
(51) Int. Cl.: A61K 6/00, A61L 24/00, A61L 27/40, A61L 24/02

(54) **IMPLANTATMATERIAL AUF BASIS EINES HYDRAULISCHEN ZEMENTS SOWIE DESSEN VERWENDUNG**
HYDRAULIC CEMENT-BASED IMPLANT MATERIAL AND USE THEREOF
MATÉRIAU D'IMPLANT À BASE DE CIMENT HYDRAULIQUE ET UTILISATION DE CELUI-CI

(30) Priorität: 06.06.2007 DE 102007027511
(43) Veröffentlichungstag der Anmeldung: 07.04.2010
(73) Patentinhaber: InnoTERE GmbH, 01445 Radebeul (DE)
(72) Erfinder: NIES, Berthold, 64407 Fränkisch-Crumbach (DE)
(74) Vertreter: Kailuweit & Uhlemann Patentanwälte
(86) Internationale Anmeldenummer: PCT/EP2008/057099
(87) Internationale Veröffentlichungsnummer: WO 2008/148878

(56) Entgegenhaltungen:
- WO-A-01/76649
- WO-A-96/39202
- JP-A- 1 111 762
- JP-A- 1 139 516
- US-B1- 6 642 285

## Beschreibung

Die Erfindung betrifft Pasten, Suspensionen oder Dispersionen zur Herstellung von Implantatmaterialien auf Basis hydraulischer Zemente, die in Form einer oder mehrerer, mineralische und/oder organische und/oder organomineralische Feststoffe enthaltender Pasten, Suspensionen oder Dispersionen vorliegen. Die Pasten, Suspensionen oder Dispersionen reagieren bei Kombination oder Reaktion mit einer wässrigen Flüssigkeit in einer zementartigen Abbindereaktion zu einem Feststoff. Die Erfindung betrifft auch die Verwendung der Implantatmaterialien als technische, medizintechnische und/oder pharmazeutische Produkte, insbesondere als Knochenzemente, Knochenersatzmaterialien, Knochenkleber, Zahnfüllmaterialien und implantierbare Wirkstoffträger.

Mineralische Reaktivsysteme sind seit langer Zeit in Form von verschiedenartigen Zementen, Gips etc. gebräuchlich. Typischerweise bestehen sie aus mineralischen Pulvern, die mit Wasser angemischt werden. Diese Mischungen binden in Minuten bis zu Tagen ab und erreichen ihre endgültige Zusammensetzung häufig erst nach Tagen bis Monaten. Die Abbindereaktion besteht üblicherweise in einer Lösung von wasserlöslichen Pulverkomponenten und anschließender Ausfällung einer stabileren oder schwerer löslichen Phase oder Salzform, bzw. in einer Umkristallisation von metastabilen Pulverkomponenten zu einer unter den Anwendungsbedingungen thermodynamisch stabilen Modifikation. Häufig reagieren auch zwei oder mehr wasserlösliche Pulverbestandteile zu einer schwerlöslichen Substanz.

Mineralische Zemente werden selten in reiner Form eingesetzt, sondern enthalten meist große Anteile an mineralischen oder organischen Füllstoffen und/oder sind durch Zusatz von organischen, mineralischen oder metallischen Fasern (oder sonstigen Verstärkungselementen) verstärkt.

Die Verbreitung mineralischer Reaktivsysteme hat in den letzten Jahrzehnten stark zugenommen, und das trotz der Zunahme und Verbreitung alternativer Materialien, wie Metallen, Keramiken und Kunststoffen. Wesentlicher Grund ist die starke anwendungsbezogene Diversifizierung der relativ geringen Anzahl prinzipiell unterschiedlicher Reaktivsysteme. Diese Diversifizierung zeigt sich vor allem in der anwendungsbezogenen Vorformulierung von Reaktivkomponenten mit Füllstoffen, Verstärkungselementen, Substanzen, die Fließfähigkeit, Adhäsion, Abbindekinetik, Frostresistenz etc. beeinflussen. Beispiele sind neben dem klassischen Portlandzement und Gips Formulierungen, die bereits auf die Verwendung als Estrich oder Putz mit einer geeigneten Art und Menge Sand abgemischt sind. Abmischungen mit verschiedenen Polymeren (gelöst und/oder dispergiert) haben den mineralischen Reaktivsystemen neue Anwendungsfelder erschlossen (z. B. Dünnbett-Fliesenkleber, injizierbare Dübelmassen), die mit den rein mineralischen Ausgangsmaterialien nicht realisierbar wären und haben dadurch die Wertschöpfung der Industrie deutlich erhöht.

In der Medizin(technik) finden mineralische Zemente ebenfalls seit einigen Jahren vermehrt Anwendung. Zunächst wurden sie als dentale Füllmaterialien (z. B. Wurzelfüllungen) und seit ca. 1990 auch als Füll- und Verstärkungsmaterialien für Knochendefekte in der Mund- Kiefer - und Gesichtschirurgie (MKG-Chirurgie) und Orthopädie verwendet. Gerade im medizinischen Bereich steht die Anwendung mineralischer Reaktivsysteme noch in den Anfängen, vor allem, weil nur eine sehr begrenzte Auswahl an Substanzen zur Anwendung im Körper akzeptabel ist und weil an Reinheit und Qualität der Produkte sehr hohe Anforderungen gestellt werden müssen. Bei den Ausgangsstoffen sind zudem viele Substanzen nicht in der geforderten Qualität erhältlich und müssen für diese Anwendungen speziell hergestellt werden.

Eine zusätzlich bestehende Hürde für die Ausdehnung des Einsatzes mineralischer Reaktivsysteme in Medizin und Technik ist die typische Anmischung aus Pulver und Flüssigkeit. Sie ist in jedem Fall aufwendig und die Qualität des Ergebnisses obliegt der Geschicklichkeit und Sorgfalt des Anwenders. Für medizinische Anwendungen kommt erschwerend hinzu, dass die dort bisher eingeführten mineralischen Zemente sehr empfindlich auf Abweichungen des Pulver/Flüssigkeitsverhältnisses reagieren und die klinischen Anwender - anders als in der Technik (z. B. im Bauwesen) - in der Zubereitung der mineralischen Reaktivsysteme bisher wenig geschult und erfahren sind (die vorhandene langjährige Erfahrung mit polymerbasierten Reaktivsystemen - PMMA-Knochenzement - ist zudem eher hinderlich, weil sich die mineralischen Reaktivsysteme völlig anders verhalten).

Die Bemühungen zur Vereinfachung der Pulver-Flüssigkeitsmischungen waren bisher nicht von durchschlagendem Erfolg gekrönt. In der Medizintechnik wurden vor allem verschiedene Mischsysteme entwickelt, die die Variabilität durch den Anwender reduzieren und den Komfort für das Anwendungspersonal erhöhen sollten.

EP1520562 A2 offenbart eine Vorrichtung zum Mischen und Austragen von flüssigen und pulverförmigen Materialien für die medizinische Verwendung mit einem Mischzylinder und mit einem durchbrochenen Mischkolben, der in dem Mischzylinder mittels einer Betätigungsstange axial und drehbar beweglich ist, mit einem axial verschiebbaren Austragskolben und mit einem verschließbaren Austragskolben am Mischzylinder. Diese Patentanmeldung zeigt beispielhaft den apparativen Aufwand, der von einem Hersteller von Knochenzementen als Lösung für die Problematik der Pulver/Flüssigkeitsmischung unter OP-Bedingungen vorgeschlagen wird. Grundsätzlich wird durch diesen Vorschlag (ebenso wie durch andere) das Problem aber nicht gelöst, sondern nur durch erhöhten apparativen Aufwand bis zu einem gewissen Grad kompensiert. Weiterhin zeigt die Praxis, dass trotz aufwendiger Apparaturen diese Vorrichtungen keineswegs ohne intensive Schulung und Übung zu zufriedenstellenden Ergebnissen führen. Eine apparative Lösung scheidet daher aus.

US 2004/244651 A1 beschreibt Calciumphosphat-Zement-Zusammensetzungen aus mindestens zwei reaktiven Komponenten in flüssiger oder pastöser Form, die als Trägerflüssigkeit ausschließlich Wasser und/oder wässrige Lösungen enthalten. Die Zusammensetzungen der beispielhaft genannten Zemente orientieren sich zwar an gebräuchlichen Calciumphosphat-Zementen, enthalten aber zusätzliche mineralische Bestandteile, um so die erzeugten Pasten zu stabilisieren. Jedem Experten auf dem Gebiet der Calciumphosphat-Zemente wird es zudem fraglich erscheinen, ob eine der beispielhaft genannten Rezepturen in der beschriebenen Zusammensetzung über einen längeren Zeitraum lagerstabil ist. Für die genannten metastabilen Calciumphosphate - TetraCalciumphosphat (TTCP), ß-Tri-Calciumphosphat (β-TCP) und α-Tri-Calciumphosphat (α-TCP) - gilt jedenfalls, dass sie nur unter praktisch wasserfreien Bedingungen stabil sind. Alle Hersteller entsprechender Zemente legen daher auch großen Wert auf Herstellbedingungen und Verpackungen, die den Zutritt von Wasser (oder Luftfeuchtigkeit) zum Zementpulver minimieren.

S. Takagi, S. Hirayama, A. Sugawara and L.C. Chow beschreiben in ihrer Arbeit PREMIXED CALCIUM PHOSPHATE CEMENT PASTES (Sixth World Biomaterials Congress Transactions; 2000) Untersuchungen zur Mischung von verschiedenen Calciumphosphat-Zementen mit Glycerin als Trägerflüssigkeit. Nach Einbringung dieser Präparationen in wässrige Lösungen reagierten sie zu Feststoffen, die allerdings relativ geringe Festigkeitswerte aufweisen und sehr lange Aushärtezeiten zeigten. Glycerin ist zudem eine sehr hygroskopische Substanz, die dazu neigt, Wasser aufzunehmen, womit die Lagerstabilität eingeschränkt ist. Dieses Problem gilt prinzipiell für alle im chemischen Sinn mit Wasser mischbaren Flüssigkeiten, die zudem in den meisten Fällen nur mit großem Aufwand wasserfrei zu bekommen sind. Die gleiche Autorengruppe beschreibt in einer neueren Publikation (Premixed rapid setting calcium phosphate composites for bone repair; Carey, Xu, Simson, Takagi, Chow in Biomaterials 26 (2005) 5002-5014) weiterentwickelte Formulierungen, die ebenfalls wasserlösliche Trägerflüssigkeiten verwenden und einen Teil der oben genannten Nachteile durch Modifikationen der Zusammensetzung teilweise kompensieren. Die Nachteile der potenziell geringen Lagerstabilität und die mangelnde universelle Anwendung auf die verschiedensten reaktiven organomineralischen Reaktivsysteme bleiben davon allerdings unberührt.

WO 2002/062721 A1 und WO 2004/093734 A2 offenbaren eine vorgemixte Calciumphosphatzementpaste aus einer Mischung aus Glycerin als Trägerflüssigkeit und einem Calciumphosphat mit verschiedenen Zusätzen, die im Kontakt mit wässrigen Lösungen zu einem Feststoff aushärten können. Diese Formulierung soll die oben genannten Nachteile der Zementpaste aus Calciumphosphaten und Glycerin durch Zusätze an Säuren und Gelbildnern reduzieren und insbesondere zu verkürzten Abbindezeiten und einer verbesserten Pastenkohäsion führen. Der prinzipielle Nachteil von Zementpasten, die auf wasserlöslichen Trägerflüssigkeiten basieren (s. o.), wird dadurch aber nicht ausgeräumt. Die Zusatzstoffe können diese nur teilweise kompensieren. Eine universell anwendbare Methode und Zusammensetzung für die Herstellung lagerstabiler pastenbasierter (Knochen-) Zemente ist damit nicht möglich.

US 6,642,285 B1 beinhaltet eine Calciumphosphat-Zement-Zusammensetzung, die eine hydrophobe Flüssigkeit enthält. Danach mischt man zunächst Calciumphosphat-Pulver mit den entsprechenden wässrigen Anmischlösungen und vermischt die entstehenden Pasten wiederum mit Ölen, um unter Zusatz von Emulgatoren und in Abhängigkeit von der Rührintensität Emulsionen zu erhalten, die nach Aushärtung der Calciumphosphat-Paste eine poröse Zementstruktur ergeben. Offenbar haben die so erhaltenen porösen Formkörper und Granulate nur eine geringe Festigkeit, so dass eine nachfolgende thermische Behandlung zu deren Verfestigung notwendig wird. Als zementartige Präparationen sind diese Zusammensetzungen und die beschriebenen Verfahren zur Herstellung ungeeignet, weil sie neben geringen mechanischen Festigkeiten noch deutlich schwieriger in der Herstellung sind, als die Pulver-/Flüssigkeitsmischungen selbst.

WO 01/76649 A1 beschreibt eine Zumischung hydrophober Flüssigkeit, insbesondere Öl oder Fett zu pulverförmigen Komponenten mineralischer Knochenzemente mit dem Ziel, die Rheologie des Knochenzements zu verbessern und das Einmischen von pharmakologischen Wirkstoffen zu erleichtern. Insbesondere soll die hydrophobe Flüssigkeit die Freisetzung pharmazeutischer Wirkstoffe (z. B. Vitamin E) im Körper verbessern. Im Ergebnis der Zumischung von maximal 10% (und bevorzugt 2-6%) der hydrophoben Flüssigkeit bleibt nach WO 01/76649 A1 ein Pulver erhalten, das vor Injizierung sorgfältig und effizient gemischt werden muss.

Es ist Aufgabe der Erfindung, Implantatmaterialien in Form leicht verarbeitbarer Pasten, Suspensionen oder Dispersionen zu schaffen, die unter Normalbedingungen über einen langen Zeitraum lagerstabil sind und vielseitig eingesetzt werden können, insbesondere auch als Knochenzemente, Knochenersatzmaterialien, Knochenkleber, Zahnfüllmaterialien und implantierbare Wirkstoffträger.

Erfindungsgemäß wird die Aufgabe durch eine Paste, Suspension oder Dispersion zur Herstellung eines Implantatmaterials auf Basis eines hydraulischen Zements aus mindestens einer Komponente in Form einer Paste, Suspension oder Dispersion gemäß Anspruch 1 gelöst. Die mindestens eine Komponente enthält wenigstens einen Calcium- und/oder Magnesiumverbindungen-haltigen pulverförmigen und reaktiven Feststoff und bindet bei Vermischung mit einer wässrigen Flüssigkeit oder nach Einbringung in eine wässrige Flüssigkeit zu einem Festkörper ab. Erfindungsgemäß enthält dabei die mindestens eine Komponente einen Feststoff und eine Trägerflüssigkeit, die substantiell wasserfrei und im chemischen Sinn nicht mit Wasser mischbar ist. Erfindungsgemäß übersteigt die Konsistenz der Paste, Suspension oder Dispersion unter Normalbedingungen nicht die einer knetbaren Masse.

Calcium- und/oder Magnesiumverbindungen-haltige pulverförmige und reaktive Feststoffe sind Feststoffe, die bei Vermischung mit einer wässrigen Flüssigkeit oder nach Einbringung in eine wässrige Flüssigkeit in einer hydraulischen Zementreaktion zu einem Festkörper abbinden können.

Die erfindungsgemäßen Implantmaterialien in Form einer oder mehrerer, mineralische und/oder organische und/oder organomineralische Feststoffe enthaltender Pasten, Suspensionen oder Dispersionen in einer ersten Flüssigkeit sind so formuliert, dass die Pasten, Suspensionen oder Dispersionen über ausgedehnte Zeiträume bei Normalbedingungen (Normalbedingungen sind definiert als 25°C und 101,3 kPa.) lagerstabil sind und dass sie bei Kombination mit einer wässrigen zweiten Flüssigkeit oder nach Einbringung in eine wässrige zweite Flüssigkeit in einer zementartigen Abbindereaktion reagieren und zu einem Festkörper abbinden. Dabei ist die erste Flüssigkeit, die Trägerflüssigkeit der mineralischen Paste, Suspension oder Dispersion, substantiell wasserfrei und im chemischen Sinne nicht mit Wasser mischbar bzw. nicht oder nur geringfügig in Wasser löslich.

Die Mischbarkeit mit Wasser im chemischen Sinn bedeutet eine homogene Verteilung der Trägerflüssigkeit in Wasser auf molekularer Ebene, die Moleküle liegen somit in der Mischung mit Wasser als Einzelmoleküle vor. Dies trifft beispielsweise zu für Glycerin, niedermolekulare ein- und zweiwertige Alkohole, Aceton, flüssige Polyethylenglykole, usw. Im Gegensatz dazu stehen Flüssigkeiten, die nicht im chemischen Sinn mit Wasser mischbar sind und/oder nur gering in Wasser löslich sind. Beispiele sind die typischen Öle (auch wenn die umgangssprachlich als Öle bezeichneten Flüssigkeiten keine einheitliche Substanzklasse darstellen). Als Grenze der Löslichkeit im Sinne der Erfindung wird eine maximale Löslichkeit der Trägerflüssigkeit in Wasser von 25% angesetzt, weil dieser Wert lösliche und unlösliche Flüssigkeiten gut differenziert. In der Praxis liegt die Löslichkeit der in einer erfindungsgemäßen Paste, Suspension oder Dispersion enthaltenen Flüssigkeiten wesentlich niedriger. Die chemische Mischbarkeit ist von der physikalischen Mischbarkeit eindeutig abzugrenzen, da im Falle physikalischer Mischbarkeit keine molekulare Verteilung der Flüssigkeiten vorliegt, sondern die gemischten Flüssigkeiten als Aggregate vorliegen, beispielsweise als Tröpfchen der einen Flüssigkeit in einer kontinuierlichen Phase der anderen Flüssigkeit. Typisches Beispiel sind Emulsionen von Ölen in Wasser und umgekehrt.

Die Mischbarkeit von Flüssigkeiten ist stark von den Umgebungsbedingungen, insbesondere der Temperatur, abhängig. Im Sinne der Erfindung sind als Umgebungsbedingungen die für die Anwendung der erfindungsgemäßen Paste, Suspension oder Dispersion relevanten Bedingungen maßgebend, das sind die üblicherweise als Normalbedingungen bezeichneten Werte von 25°C und 101,3 kPa.

Die Konsistenz der Paste, Suspension oder Dispersion übersteigt unter Normalbedingungen nicht die Konsistenz einer knetbaren Masse. Charakteristisch ist in jedem Fall, dass die erhaltene Paste, Suspension oder Dispersion unter Normalbedingungen eine flüssige bis pastöse Konsistenz hat, je nachdem, welche Trägerflüssigkeit als Dispersionsmittel zu Einsatz kommt und in welcher Zusammensetzung, Partikelgröße, Partikelform und Konzentration die dispergierten Feststoffe vorliegen und inwiefern die Konsistenz durch weitere Zusatzstoffe gezielt eingestellt wurde.

Die Spannweite der mindestens einen Komponente reicht dabei von der Konsistenz relativ dünnflüssiger Dispersionsfarben bis hin zu einer Konsistenz von hochgradig strukturviskosen Pasten, Knetmassen und Kitten. Die Konzentration der dispergierten Feststoffe ist dabei nur eine Möglichkeit, die Konsistenz zu beeinflussen, wie die Tatsache zeigt, dass beispielsweise Dispersionsfarben sehr hohe Feststoffgehalte aufweisen können und trotzdem eine vergleichsweise niedrige Viskosität haben. Ähnlich verhält es sich mit erfindungsgemäßen Dispersionen von Calciumphosphat-Zementen in dünnflüssigen Ölen (z. B. Miglyol 812) und dem Zusatz von Tensiden, die auch bei einem Feststoffgehalt von > 75% unter intensiver Vermahlung auf die Konsistenz von Sirup eingestellt werden können. Ohne damit einschränkend auf die Ausgestaltung der Erfindung zu sein, wird die Konsistenz der Dispersion vor allem dann bevorzugt auf die Konsistenz von Sirup oder Zahnpasta eingestellt, wenn die Verwendung als 2-Komponentensystem und in einer 2-Kammer-Spritze vorgesehen ist. Eine vergleichbare Konsistenz wird bevorzugt auch dann gewählt, wenn die Dispersion als Ein-Komponentensystem vorgesehen ist, das mittels einer Spritze ausgetragen werden soll. Eine wesentlich höhere Viskosität wird vorzugsweise dann gewählt, wenn die Dispersion als Ein-Komponentensystem vorgesehen ist, das in offener Form angewendet werden soll. Im medizinischen Bereich kann das beispielsweise der Ersatz von Knochenwachs sein oder die Verwendung als Knochenfüllstoff bei offener Implantation. Hier ist eine hohe Viskosität ggf. mit der Ausprägung einer starken Strukturviskosität besonders bevorzugt. In Bezug auf die Konsistenz veranschaulichende Vergleichsmaterialien sind Modelliermassen, Kneten, Kitte, etc.

Kern der vorliegenden Erfindung ist die Formulierung reaktiver mineralischer oder organomineralischer Zementkomponenten in Trägerflüssigkeiten, die wasserfrei und im chemischen Sinne nicht mit Wasser mischbar sind. Erfindungsgemäß sind die Trägerflüssigkeiten nicht oder nur gering (< 25%) in Wasser löslich, bzw. Wasser ist nur gering (< 25%) in den Trägerflüssigkeiten löslich. Die (organo-) mineralischen Pulver werden vorzugsweise in ihrer endgültigen Mischung in diesen nicht-wässrigen Flüssigkeiten zu einer Paste suspendiert und sind in dieser Form lagerfähig, sofern die Pulver fein dispergiert sind und eine geeignete Trägerflüssigkeit gewählt wurde. Ggf. sind je nach Zement spezielle Herstellschritte und Maßnahmen - insbesondere intensive Vermahlung, Zusatz von Tensiden, Zusatz von Polymeren, etc. - sinnvoll, um die Stabilität der Dispersion auf Dauer zu gewährleisten.

Die in der wasserfreien und mit Wasser nicht mischbaren Flüssigkeit dispergierten, reaktiven und hydraulisch abbindbaren Feststoffe reagieren, sobald die erhaltenen Pasten Suspensionen oder Dispersionen mit einer geeigneten wässrigen Lösung vermischt werden oder in eine wässrige Flüssigkeit eingebracht werden. Diese Tatsache ist besonders überraschend, da die in der wasserfreien und mit Wasser nicht mischbaren Flüssigkeit dispergierten Feststoffe zunächst in die wässrige Phase übertreten müssen, bevor sie miteinander reagieren können. Die Zusammensetzung der wässrigen Lösung als Medium und/oder Reaktionspartner für die Abbindereaktion lehnt sich dabei im Falle von 2-Komponentensysteme an die Zusammensetzung an, die auch bei konventioneller Anmischung der Zemente verwendet wird. Prinzipiell kann die Zusammensetzung der wässrigen Lösung aber weitgehend frei auf den vorgesehenen Anwendungszweck abgestimmt werden, indem beispielsweise die Konzentration der gelösten Substanzen so eingestellt wird, dass die bevorzugte Abbindegeschwindigkeit erhalten wird. Wichtig ist vielmehr, dass alle mit Wasser (oder mit oder in Wasser untereinander) reaktiven Komponenten der eingesetzten Bestandteile des hydraulischen Zements in der wasserfreien und mit Wasser nicht mischbaren Flüssigkeit dispergiert sind. Daraus folgt, dass entsprechend alle anderen Bestandteile des Zements auch oder bevorzugt in der wässrigen Lösung enthalten sein können. Die wässrige Lösung kann dementsprechend in einer 2-Komponentenversion die Konsistenz einer zweiten Paste annehmen.

Üblicherweise benötigen bekannte mineralische oder organomineralische Reaktivsysteme sowie Implantatmaterialien auf Basis hydraulischer Zemente Wasser als Medium und/oder Reaktionspartner für die Abbindereaktion. In vielen Fällen enthält die typische Flüssigkeit bei Pulver-/Flüssigkeitssystemen außer Wasser noch gelöste oder suspendierte organische oder anorganische Substanzen (bzw. diese lösen sich bei Kontakt mit Wasser sehr schnell aus der Pulverkomponente, so dass sie praktisch unmittelbar nach der Anmischung zur Verfügung stehen) mit denen die Kinetik der Abbindereaktion, die Kristallstruktur des Abbindeprodukts, die Viskosität der Flüssigkeit oder der Zementpaste, das Benetzungs- und Mischungsverhalten etc. beeinflusst werden. Es wird entweder die gesamte Pulverkomponente oder aber zumindest alle deren Bestandteile, die mit der wässrigen Lösung reagieren können, bzw. die in einer wässrigen Lösung miteinander reagieren können, in einer nicht-wässrigen Trägerflüssigkeit, die mit Wasser im chemischen Sinn nicht mischbar ist, zu einer Paste dispergiert, in der diese Pulverkomponenten auf längere Zeit und insbesondere > 6 Monate und bevorzugt > 2 Jahre unter Normalbedingungen lagerstabil sind. Reaktive organomineralische Zementkomponenten können grundsätzlich in wasserhaltigen Lösungen nur so formuliert werden, dass sie weitgehend unreaktiv bleiben, indem sie durch Auswahl geeigneter Stabilisatoren oder extreme pH-Werte in ihrer Reaktionsfähigkeit behindert werden. Solche Maßnahmen widersprechen aber dem Konzept der vorliegenden Erfindung, insbesondere im Hinblick auf die Verwendung als medizinische Implantatmaterialien, für die weder Stabilisatoren, die eine Mineralisierung des Knochens beeinträchtigen können noch extreme pH-Werte im sauren oder alkalischen Bereich akzeptabei sind. Es werden daher ausschließlich Trägerflüssigkeiten für die Herstellung organomineralischer Pasten berücksichtigt, die praktisch wasserfrei sind, um vor allem neuartige Formulierungen zu beschreiben, die in ihrer prinzipiellen Auslegung universell auf verschiedenartige Implantatmaterialien als organomineralische Reaktiv-systeme anwendbar sind.

Bevorzugt sind Pasten, Suspensionen oder Dispersionen zur Herstellung von Implantatmaterialien, bei denen die organomineralischen Feststoffe als feine Pulver suspendiert/dispergiert sind, da es ein Ziel der Entwicklung ist, möglichst wenig Trägerflüssigkeit für die Herstellung der Pasten, Suspension oder Dispersion zu benötigen, um so entsprechend viel Freiraum für die anwendungsspezifische Formulierung des Implantatmaterials zu erhalten. Dabei hat es sich als besonders vorteilhaft erwiesen, wenn für die Herstellung der Pasten, Suspension oder Dispersion eine Vermischung mit hohem Energieeintrag benutzt wird. Bevorzugt werden daher insbesondere Formulierungen, in denen der Feststoffgehalt der Paste in der Flüssigkeit bei > 2:1 und besonders bevorzugt bei > 3:1 (Masse des Pulvers : Masse der Trägerflüssigkeit) liegt und wobei > 10% der Feststoffe (Masse) eine Partikelgröße von < 10 µm haben. Zudem zeigen Versuche, dass besonders vorteilhafte Eigenschaften der Zemente und besonders hohe Feststoffgehalte der Pasten zu erzielen sind, wenn eine breite Partikelgrößenverteilung erzielt wird, insbesondere wenn > 10% der Feststoffe aus Partikeln < 10 µm und wenn > 10% der Feststoffe aus Partikeln > 50 µm bestehen.

Bei der Auswahl der (organo-) mineralischen Zemente, die für die erfindungsgemäß herstellbare Implantatmaterialien geeignet sind, gibt es praktisch keine Einschränkungen, da letztlich alle auf dem gleichen Prinzip beruhen, nämlich aus (organo) mineralischen Pulvern und wässrigen Lösungen oder reinem Wasser angemischt werden. Sowohl Gips, als auch silikatische Zementpulver lassen sich in gleicher Weise in wasserfreien Trägerflüssigkeiten dispergieren, wie z. B. Calciumphosphate. Insbesondere gilt das für die verschiedensten Öle als Trägerflüssigkeiten. In Abgrenzung von organischen, insbesondere polymeren Zementsystemen, sind die Implantatmaterialien dadurch charakterisiert, dass die zur Abbindung und Ausbildung eines festen Stoffes führende Reaktion im wesentlichen anorganische Reaktionspartner einschließt und dass diese Reaktionspartner aus der Gruppe der Silikate, Phosphate, Sulfate, Carbonate, Oxide und Hydroxide und/oder deren Mischungen bestehen. Wie bereits weiter oben erwähnt, enthalten handelsübliche Zemente zuweilen große Mengen an Füllstoffen, die nur oberflächlich an der Reaktion teilnehmen (also insbesondere in die Zementmatrix eingebunden werden), aber nur mittelbar an der Abbindereaktion beteiligt sind. Die Teilnahme kann beispielsweise auch darin bestehen, dass ein Füllstoff als Kristallisationskeim für die Mineralisation während der Abbindereaktion fungiert und damit insbesondere die Abbindekinetik beeinflusst, aber selbst sonst nicht umgesetzt wird. Dies gilt beispielsweise im Falle der Calciumphosphat-Zemente für den Zusatz an gefälltem Hydroxylapatit. Für technische Zemente werden kostengünstige Zusätze vielfach auch aus ökonomischen Gründen zugesetzt. Vorzugsweise enthalten die Pasten, Suspensionen oder Dispersionen zur Herstellung von Implantatmaterialien bezogen auf die dispergierten mineralischen Feststoffe zu > 30% Salze der Kieselsäure oder deren Kondensate und/oder Salze der Phosphorsäure oder deren Kondensate und/oder Salze der Schwefelsäure und/oder Salze der Kohlensäure oder deren Mischungen, bzw. Oxide oder Hydroxide.

Als Kationen der Salze der vorgenannten Säuren kommen vor allem Calcium- und Magnesium-Ionen in Betracht. Für technische Anwendungen können noch unterschiedliche Mengen an anderen Salzen hinzukommen. Besonders bevorzugt sind insbesondere für die medizinischen Anwendungen Pasten, Suspensionen oder Dispersionen zur Herstellung von Implantatmaterialien, bei denen die dispergierten Feststoffe zu > 75% aus Calcium- und/oder Magnesiumsalzen bzw. deren Oxiden und/oder Hydroxiden bestehen.

Eine weitere Spezifizierung speziell für medizinische Anwendungen besteht in der bevorzugten Verwendung von Calcium- und Magnesiumsalzen der Ortho-Phosphorsäure. In handelsüblichen und besonders in experimentellen Calciumphosphat-Zementen sind bereits praktisch alle vorkommenden Calcium- und Magnesiumsalze der Phosphorsäure eingesetzt worden (Calciumphosphat-Zemente umfassen hier auch solche, die in erheblicher Menge Magnesiumsalze enthalten). Eingeschlossen sind hier auch solche Ca- und Mg-Salze, - Oxide und -Hydroxide, die weitere Ionen enthalten, insbesondere Na, K, NH₄ und/oder solche die gleichzeitig Ca und Mg enthalten. Eingeschlossen sind auch ausdrücklich die Ca - und Mg-Salze der Glycerophosphorsäure und die anderer mono- oder di-substituierter organischer Phosphorsäureester. Es werden Pasten, Suspensionen oder Dispersionen zur Herstellung von Implantatmaterialien bevorzugt, bei denen die suspendierten mineralischen Feststoffe zu > 50% aus Calcium- und/oder Magnesiumphosphaten bestehen. Daneben können die Feststoffe übliche Füllstoffe, wie z. B. CaCO₃und/oder SrCO₃ enthalten.

Besonders bevorzugte Calciumverbindungen sind Monocalciumphosphat Monohydrat (MCPM), Monocalciumphosphat Anhydrit (MCPA), Dicalciumphosphat Anhydrit (DCPA), Dicalciumphosphat Dihydrat (DCPD), Octacalciumphosphat (OCP), α-Tricalciumphosphat (α-TCP), ß-Tricalciumphosphat (ß-TCP), amorphes Calciumphosphat (ACP), Hydroxylapatit (HA), Calcium-defizientes Hydroxylapatit (CdHA), substituiertes Hydroxylapatit, nichtstöchiometrisches Hydroxylapatit, nano Hydroxylapatit, Tetracalciumphosphat (TTCP), Calciumsulfat (CaSO₄), Calciumsulfat Hemihydrat (CaSO₄x0,5H₂O), Calciumsulfat Dihydrat (CaSO₄x2H₂O), Calciumoxid (CaO), Calciumhydroxid (Ca(OH)₂), Calciumcarbonat (CaCO₃), Calciumglycerophosphat, Calciumcitrat, Calciumlactat, Calciumacetat, Calciumtartrat, Calciumchlorid (CaCl₂), Calciumsilikate und/oder deren Mischungen untereinander und/oder mit anderen Substanzen, deren Anteil < 5 Gew.-% beträgt.

Besonders bevorzugte Magnesiumverbindungen sind Magnesiumhydrogenphosphat (MgHPO₄) in Form der Hydrate und als wasserfreie Substanz, Trimagnesiumphosphat (Mg₃(PO4)₂), Magnesiumdihydrogenphosphat (Mg(H₂PO₄)₂) in Form der Hydrate und als wasserfreie Substanz, Magnesiumchlorid (MgCl₂) in Form der Hydrate und als wasserfreie Substanz, Magnesiumglycerophosphat, Magnesiumhydroxid (Mg(OH)₂), Magnesiumhydroxidcarbonat (z. B. als 4MgCO₃xMg(OH)2x5H₂O), Magnesiumoxid (MgO), Magnesiumcitrate (Mg₃(C₆H₅O₇)₂ oder Mg(C₆H₆O₇), Calcium-Magnesiumcarbonat (CaMg(CO₃)₂, Dolomit) und/oder deren Mischungen untereinander (und/oder mit anderen Substanzen, deren Anteil < 5 Gew.-% beträgt).

Aus der Vielzahl von möglichen und in der Literatur beschriebenen Calciumphosphat-Zementen (CP-Zemente) sind besonders solche bevorzugt, die auf der Verwendung metastabiler Calciumphosphate basieren, die sich unter geeigneten Umgebungsbedingungen oder Reaktionsbedingungen allein oder mit Reaktionspartnern spontan in eine der thermodynamisch stabileren Formen - Hydroxylapatit, Ca-defizientes Hydroxylapatit, substituiertes Hydroxylapatit oder Calciumhydrogenphosphat - umwandeln. Vor allem diese CP-Zemente binden unter Bedingungen ab, die dem biologischen System des Knochens noch zuträglich sind. Beschrieben sind auch Zementsysteme, die kristalline Phosphorsäure oder Calciumoxid enthalten und damit zumindest kurzfristig in der Umgebung extreme pH-Werte von < 4 oder > 10 erzeugen, wobei insbesondere die niedrigen Extreme dem Knochen abträglich sind. Grundsätzlich sind diese Darreichungsformen auch für die letzteren (extremen) Zementformulierungen besser geeignet als die konventionellen Formen, da der Reaktionsraum enger begrenzt und der Abstand der Reaktionspartner voneinander besser definiert werden kann. Besonders bevorzugt sind CP-Zemente, die als Hauptbestandteile MCPM, MCPA, DCPA, DCPD, OCP, α-TCP, β-TCP, ACP, HA, CdHA, substituiertes HA, nicht stöchiometrisches HA, nano HA, TTCP, CaSO₄, CaSO₄x0,5H₂O, CaSO₄x2H₂O, CaO, Ca(OH)₂ oder CaCO₃ oder deren Mischungen enthalten. Für die medizinische Anwendung sind Pasten, Suspensionen oder Dispersionen zur Herstellung von Implantatmaterialien ganz besonders bevorzugt, bei denen die suspendierten mineralischen Feststoffe zu >25% aus der α- oder ß-Modifikation des Tricalciumphosphats (TCP), aus TTCP (Tetracalciumphosphat), DCPA (Dicalciumphosphat Anhydrit) oder ACP (amorphem Calciumphosphat) bestehen.

In der neueren Literatur sind zudem für den medizinischen Einsatz auch Zemente beschrieben, die in der Kristallstruktur des Struvits aushärten, bzw. Struvit als einen bestimmenden Anteil des ausreagierten Zements enthalten (EP 1296909 B1 Magnesium-Ammonium-Phosphat-Zemente, deren Herstellung und Verwendung). Diese Magnesium-Ammonium-Phosphat-Zemente enthalten typischerweise erhebliche Anteile an Calciumphosphaten und stellen eine besondere Ausführungsform der Implantatmaterialien dar. Für diese Struvit-Zemente wird beschrieben, dass sie eine sehr gute Kohäsion haben und relativ schnell nach Anmischung einen steilen Festigkeitsanstieg aufweisen (Schwardt et al., Kyphos FS™ Calcium Phosphate for Balloon Kyphoplasty: Verification of Compressive Strength and Instructions for Use. European Cells and Materials Vol. 11. Suppl. 1. 2006 (page 28)). Vorzugsweise ist die Kombination Magnesium-Ammonium-Phosphat-basierter Pasten, Suspensionen oder Dispersionen zur Herstellung von Implantatmaterialien mit hydrophoben, mit Wasser nicht mischbaren Flüssigkeiten als Paste eine weitere bevorzugte Ausführungsform der Erfindung. Dazu enthalten die Feststoffe auch Ammoniumsalze, die mit einer der Magnesiumverbindungen zu einer Magnesium-Ammonium-Phosphat-Festsubstanz abbinden können. Die Ammoniumsalze sind dabei ausgewählt aus di-Ammoniumhydrogenphosphat, Ammonium di-Hydrogenphosphat, Ammoniumsulfat und Ammoniumacetat und wasserlöslichen Ammoniumsalzen, die bioverträgliche Anionen enthalten.

Sowohl technische als auch medizinische mineralische Reaktivsysteme enthalten häufig Füllstoffe. Für die medizinischen Anwendungen sind besonders solche Füllstoffe wichtig, die nicht unmittelbar an der Zementreaktion beteiligt sind, sondern dazu beitragen, die Zementreaktion unter weitgehend physiologischen Bedingungen ablaufen zu lassen. In besonderer Weise trifft dies für die Hydrogenphosphate des Ca und Mg zu, sowohl für die Hydrate, als auch die wasserfreien Substanzen. Diese Hydrogenphosphate zeigen wegen ihrer höheren Löslichkeit als der Tricalciumphosphate und der ausgeprägten Pufferwirkung im neutralen Bereich eine starke Wirkung zur Dämpfung eventuell bei der Abbindereaktion auftretender Extreme bei pH-Wert oder Ionenkonzentration. Nach einer weiteren Ausgestaltung enthalten die Pasten, Suspensionen oder Dispersionen zur Herstellung von Implantatmaterialien als mineralische Feststoffe zu > 5% Calcium- oder Magnesiumhydrogenphosphat (CaHPO₄ oder MgHPO₄) als Hydrat oder wasserfreie Substanz. Die Hydrogenphosphate des Mg und Ca spielen in dieser Hinsicht eine Sonderrolle, weil sie längerfristig ebenfalls umgesetzt werden; im Falle der Struvit-Zemente in Kombination mit Ammoniumsalzen ist das Magnesiumhydrogen-phosphat sogar der reaktionsbestimmende Partner. Ein weiterer wichtiger Füllstoff ist das Calciumcarbonat, das auch langfristig umgesetzt wird.

Ebenfalls für medizinische Anwendungen sind seit vielen Jahren Implantatwerkstoffe in Gebrauch, die aus Calciumsulfaten, also Gips bestehen. Hinzu kommen seit einigen Jahren auch Entwicklungen, die außer Calciumsulfaten auch Calciumphosphate enthalten (PCT/SE99/02475). Die Calciumsulfate dienen dabei in der Regel als Bindemittel für die Calciumphosphate, die in diesem Fall üblicherweise unreaktiv sind, also selbst keine Abbindereaktion zeigen. Allerdings liegen auch Literaturberichte vor, in denen Zemente aus reaktiven Calciumphosphaten und Calciumsulfaten hergestellt wurden. In beiden Fällen sind die erfindungsgemäßen Implantatmaterialien ohne Einschränkung anwendbar. Es sind Pasten, Suspensionen oder Dispersionen zur Herstellung von Implantatmaterialien bevorzugt, bei denen die suspendierten mineralischen Feststoffe zu > 10% Calciumsulfat und insbesondere zu > 10% Calciumsulfat-Hemihydrat enthalten.

Von den verschiedenen CP-Zementen für medizinische Anwendungen sind besonders solche interessant und bevorzugt, die als Reaktionsprodukt eine ähnliche Zusammensetzung wie die der Knochenmineralien ergeben. Es sind daher Pasten, Suspensionen oder Dispersionen zur Herstellung von Implantatmaterialien bevorzugt, bei denen das Endprodukt der zementartigen Abbindereaktion mit einer wässrigen Lösung oder reinem Wasser zu > 50% Hydroxylapatit, Calcium defizienter Hydroxylapatit, substituierter Hydroxylapatit und/oder nanokristalliner Hydroxylapatit oder deren Mischung ergibt und wobei das Endprodukt ein molares Calcium/Phosphat-Verhältnis (C/P-Verhältnis) von >1,35 aufweist. Das C/P-Verhältnis von stöchiometrischem Hydroxylapatit (HA) liegt bei 1,67, das von Cadefizientem HA bei 1,5. Die gewählte Grenze von 1,35 trägt der Tatsache Rechnung, dass auch niedrigere C/P-Verhältnisse als 1,5 noch als HA kristallisieren und dass relativ hohe Substitutionsgrade auftreten können. Bevorzugt sind daher Pasten, Suspensionen oder Dispersionen zur Herstellung von Implantatmaterialien, die in der Gesamtheit ihrer Calcium-und Phosphatverbindungen ein C/P-Verhältnis von > 1,35 aufweisen.

Beispiele für verschiedenartige Calciumphosphat Zemente, die als HA oder Ca-defizientes HA oder substituierter HA oder Nano-HA oder wenig kristalliner HA aushärten, sind in der Literatur seit ca. 1986 (Brown WE, ChowLC. A new calcium phosphate water setting cement. In: Brown PW, editor. Cements research progress. Westerville, OH: American Ceramic Society; 1986. p. 352-79.) mehrfach beschrieben. Nachfolgend einige Literaturstellen zu besonders wichtigen Calciumphosphat Zementen:
1. Chow (s.o.)
2. US000005336264A (Constanz, Norian Corporation)
3. US 6,117,456 (Lee, et al; ETEX corporation)
4. US 5,262,166 (Sung-Tsuen Liu)
5. EP1 302 453 A1 (Hirano; Mitsubishi)
6. Bohner M, Merkle HP, van Landuyt P, Trophardy G, Lemaitre J. Effect of several additives and their admixtures on the phisico-chemical properties of a calcium phosphate cement. J Mater Sci Mater Med 2000; 11 : 111-116
7. Driessens FCM, Boltong MG, Bermudez O, Planell JA, Effective formulations for the preparation of calcium phosphate bone cements. J Mater Sci Mater Med. 1994; 5: 164-170

Hydroxylapatit (HA) ist unter physiologischen Bedingungen stabil und wird daher ebenso wie die Knochenmineralien nur zellulär abgebaut. Als wesentlich leichter löslich ist Calciumhydrogenphosphat Dihydrat (DCPD) bekannt. CP-Zemente mit DCPD als Abbindeprodukt sind ebenfalls in der Literatur beschrieben und werden vor allem für Anwendungen favorisiert, in denen eine relativ schnelle Auflösung des Materials angestrebt ist. Übliche Ausgangsmaterialien für DCPD-Zemente sind β-TCP und Monocalciumphosphat. Das Reaktionsprodukt hat bei der Verwendung reiner Ausgangsmaterialien ein C/P-Verhältnis von 1. Pasten, Suspensionen oder Dispersionen zur Herstellung von Implantatmaterialien, bei denen das Endprodukt der zementartigen Abbindereaktion mit einer wässrigen Lösung oder reinem Wasser zu > 50% Calciumhydrogenphosphat ist und wobei das Endprodukt ein molares Calcium/Phosphat-Verhältnis von 0,9-1,35 aufweist, sind eine vorteilhafte Ausgestaltung der vorliegenden Erfindung. Danach weisen die Pasten, Suspensionen oder Dispersionen zur Herstellung von Implantatmaterialien in der Gesamtheit ihrer Calcium- und Phosphatverbindungen ein C/P-Verhältnis von 0,9-1,35 auf.

Unabhängig von der Zusammensetzung der reaktiven organomineralischen Substanzen als Feststoffe besteht ein Hauptaspekt der Erfindung in der Einbringung dieser Substanzen in eine nicht-wässrige Trägerflüssigkeit, wobei es sich bei dieser Trägerflüssigkeit vorzugsweise um eine organische Flüssigkeit handelt, die mit Wasser nicht im chemischen Sinne mischbar ist und in Wasser praktisch unlöslich ist und in der Wasser ebenfalls praktisch unlöslich ist. Als Grenze für die gegenseitige Löslichkeit wird dabei ein Wert von 25% (bezogen auf das Volumen) angesehen. In der Regel und bevorzugt liegt dieser Grenzwert wesentlich geringer, also etwa bei < 10% und besonders bevorzugt bei < 5%. Flüssigkeiten, auf die dies zutrifft, sind sowohl dem Laien, als auch dem Fachmann hinreichend bekannt. Für medizinische Anwendungen kommen hier insbesondere pharmazeutische, kosmetische und Lebensmittel-technische flüssige Hilfsstoffe in Betracht, auf die obige Spezifizierungen zutreffen.

Während die Flüssigkeit nicht oder nur gering in Wasser löslich ist, so enthält sie doch bevorzugt feste oder flüssige dispergierte Substanzen, die in Wasser gut löslich oder zumindest stark quellbar sind oder im chemischen Sinne mit Wasser mischbar sind. Hierbei kann es sich um reaktive Substanzen des Zements handeln, oder solche, die die Reaktivität des Zements beeinflussen, ohne in nennenswertem Umfang an der Reaktion selbst teilzunehmen. Weiterhin um Hilfsstoffe, die die Dispergierung des Zements in der organischen Flüssigkeit fördern, die Mischbarkeit der Paste mit einer wässrigen Flüssigkeit verbessern, als Wirkstoffe fungieren oder dazu dienen, im endgültigen Zement ein Porensystem zu erzeugen. Letzteres trifft insbesondere auf leicht vernetzte Polyelektrolyte zu, wie sie als Superabsorber bekannt sind und beispielsweise aus leicht vernetzter Polyacrylsäure oder Carboxymethylstärke bestehen. Insbesondere kommt diesen Substanzen die Funktion zu, sich sehr schnell in Wasser zu lösen und die Viskosität der wässrigen Phase so zu beeinflussen, dass sich eine makroskopisch homogene physikalische Mischung beider Phasen ausbilden kann.

Zur Auslösung der zementartigen Abbindereaktion der Pasten, Suspensionen oder Dispersionen zur Herstellung von Implantatmaterialien müssen die in der organischen Flüssigkeit dispergierten Feststoffe mit der/einer wässrigen Lösung oder reinem Wasser in innigen Kontakt gebracht werden, so dass sich diese Feststoffe zumindest teilweise in Wasser auflösen und umkristallisieren bzw. in der wässrigen Phase wieder präzipitieren können. Dazu muss eine innige Vermischung der Paste mit einer wässrigen Lösung im Sinne einer physikalischen Mischung (also insbesondere die Ausbildung einer Emulsion und/oder Dispersion) erfolgen, wobei sich eine möglichst große Fläche als Phasengrenze ausbildet, über die Wasser und Feststoffe miteinender in Kontakt treten können und wodurch die Auflösung der wasserlöslichen Bestandteile der Paste ermöglicht wird. Dabei ist dem Fachmann bekannt, dass die verschiedenen mit Wasser im chemischen Sinn nicht mischbaren organischen Flüssigkeiten in unterschiedlicher Weise geeignet sind mit Wasser physikalische Mischungen einzugehen. Die Ausbildung und Stabilität solcher physikalischer Mischungen hängt neben der Art der Flüssigkeiten wesentlich von der eingebrachten Energie bei der Vermischung der beteiligten Partner ab. Da bei der Vermischung der erfindungsgemäßen Pasten, Suspensionen oder Dispersionen zur Herstellung von Implantatmaterialien mit wässrigen Lösungen in der Regel keine aufwendigen apparativen Vorrichtungen für den Eintrag hoher Energiemengen zur Verfügung stehen, kommt der Auswahl der organischen Flüssigkeit eine große Bedeutung zu. Die notwendige Energie zur Vermischung der Flüssigkeiten kann durch oberflächenaktive Substanzen stark herabgesetzt werden, in besonderen Fällen soweit, dass sich Emulsionen - insbesondere Mikroemulsionen - zwischen den ansonsten nicht mischbaren Flüssigkeiten spontan bilden. Dieser Aspekt hat für die Erfindung ausschlaggebende Bedeutung, wie hier nicht näher beschriebene Versuche mit schlecht mischbaren Flüssigkeiten (Pasten und wässrigen Lösungen) gezeigt haben, bei denen eine manuelle Mischung praktisch nicht möglich war und es zu keiner Abbindereaktion bzw. Ausbildung eines Feststoffs kam. Ein wichtiger Aspekt sind daher Pasten, Suspensionen oder Dispersionen zur Herstellung von Implantatmaterialien, bei denen die als Trägermedium für die Paste dienende organische Flüssigkeit mit Wasser und/oder wässrigen Lösungen im physikalischen Sinn mischbar ist und/oder wobei die organische Flüssigkeit Substanzen enthält, die eine Ausbildung einer physikalischen Mischung mit Wasser oder wässrigen Lösungen (Bildung einer Emulsion) unterstützen und eine solche stabilisieren können. Solche Substanzen können Tenside oder hochdisperse Feststoffe sein, *siehe dazu* Monographie "Die Tenside", Kosswig/Stache, Carl Hanser Verlag München Wien, 1993, ISBN 3-446-16201-1. Zum Thema Emulsionen wird auf die Arbeit "Makro- und Mikroemulsionen" von H.-D. Dörfler: Grenzflächen und kolloid-disperse Systeme. Springer, Berlin, 2002, Kap. 13. verwiesen.

In einer besonderen Ausgestaltungsform der Erfindung wird die spontane Ausbildung von Mikroemulsionen zwischen der Paste und der wässrigen Lösung angestrebt. Im Unterschied zu Makroemulsionen, die zur Bildung üblicherweise eines Energieeintrags bedürfen und thermodynamisch nicht stabil sind, bilden sich Mikroemulsionen auch unter Umgebungsbedingungen spontan aus und sind thermodynamisch stabil. Sie enthalten neben einer wässrigen und einer organischen (Öl-) Phase mindestens ein Tensid und in der Regel eine üblicherweise als Co-Tensid oder Lösungsvermittler bezeichnete niedermolekulare organische Flüssigkeit, bei der es sich beispielsweise um einen kurz- bis mittelkettigen Alkohol handeln kann, für die aber auch viele andere organische Flüssigkeiten in Betracht kommen. Ebenso wie die Tenside oder Emulgatoren können auch die Co-Tenside oder Lösungsvermittler in der Paste oder der wässrigen Lösung vorliegen. In der Regel hängt diese Auswahl vor allem von der besseren Löslichkeit des Zusatzes in der jeweiligen Phase ab. Bevorzugt werden daher in dieser besonderen Ausführungsform Pasten, Suspensionen oder Dispersionen zur Herstellung von Implantatmaterialien bei denen die organische Flüssigkeit aus mindestens 2 Substanzen besteht, die bei Normalbedingungen flüssig sind und wobei jede der Substanzen zu mindestens 0,1% in der ersten Flüssigkeit enthalten ist. In dieser Ausführungsform gilt die Spezifizierung der geringen Wasserlöslichkeit der organischen Flüssigkeit nur für eine der beiden Substanzen, die gemeinsam die organische Flüssigkeit bilden.

Wie weiter oben bereits dargestellt, sind sehr viele organische Flüssigkeiten als Trägerflüssigkeiten für die erfindungsgemäßen Pasten, Suspensionen oder Dispersionen zur Herstellung von Implantatmaterialien geeignet. Grundsätzlich ungeeignet sind solche Flüssigkeiten, die einen sehr niedrigen Siedepunkt haben und ggf. explosionsgefährdend sind, aus toxikologischen Gründen Einschränkungen unterliegen, wegen sehr hoher Viskosität die Einarbeitung von pulverförmigen Feststoffen erschweren, nahe der Normaltemperatur bereits fest werden oder mit einem oder mehreren der organomineralischen Feststoffe unverträglich sind, bzw. unerwünschte Reaktionen eingehen können. Ungeeignet sind auch organische Flüssigkeiten, die als solche nicht lagerstabil sind oder zur Erreichung von Lagerstabilität aufwendiger Maßnahmen bedürfen. Beispielhaft seien hier die grundsätzlich sehr gut geeigneten Öle (Triglyceride) genannt, wobei in dieser Gruppe solche Vertreter als ungeeignet anzusehen sind, die ohne aufwendige Maßnahmen zum Ranzigwerden neigen. Für die bevorzugte Verwendung der erfindungsgemäßen Implantatmaterialien als implantierbare Medizinprodukte oder Arzneimittel wird auf "Fiedler - Lexikon der Hilfsstoffe" (Ausgabe 01/2002) verwiesen. Bevorzugt werden Mischungen von zwei oder mehr organischen Flüssigkeiten, über die spezielle Eigenschaften der Paste erzielt werden können. In diesem Sinne werden vor allem Pasten, Suspensionen oder Dispersionen zur Herstellung von Implantatmaterialien bevorzugt, in denen die organische Flüssigkeit zu > 50% aus aliphatischen Kohlenwasserstoffen, Estern, Ethern, weitgehend wasserunlöslichen organischen Säuren oder weitgehend wasserunlöslichen ein- oder mehrwertigen Alkoholen mit einem Molekulargewicht von < 2500 oder deren Mischungen besteht.

Besonders bevorzugt sind im Hinblick auf eine medizinische oder medizintechnische Anwendung weitgehend wasserunlösliche und untoxische organische Flüssigkeiten, die im Körper leicht abgebaut oder ausgeschieden werden können. Weiterhin bevorzugt sind solche organischen Flüssigkeiten, die unter Normalbedingungen eine niedrige Viskosität aufweisen, um so die Einarbeitung von (organo-) mineralischen Pulvern zu erleichtern. Ganz besonders bevorzugt sind die nachfolgend genannten organischen Flüssigkeiten, ihre Homologen, ihre Mischungen untereinander und mit ihren Homologen: Glycerintriacetat, Glycerintributyrat, Glycerintrioleat, Glycerindioleat, Glycerinmonooleat, Caprylcaprat, Decyloleat, Isopropylmyristat, Isopropylpalmitat, Ölsäure, Oleylalkohol, Oleyloleat, kurzkettige Triglyceride, mittelkettige Triglyceride (z. B. Myritol® 318 PH, Miglyol 810, Miglyol® 812, Miglyol® 829), kurz- und mittelkettige Fettsäureester des Propylenglycols (z. B. Miglyol® 840) Ethylbenzoylacetat, Ethylbutyrat, Ethylbutyrylacetat, Ethyloleat, Ethylcaproat, Ethylcaprylat, Ethylcaprat, Ethyllaurat, Ethyllävulinat, Ethylmyristat, Ethylpalmitat, Ethyllinoleat, Ethylstearat, Ricinolsäure, Linolsäure, Linolensäure, Arachinsäure, Oleinsäure, Ethylarachidat, α-Tocopherol, β-Tocopherol, γ-Tocopherol, δ-Tocopherol, Benzylalkohol, Benzylbenzoat, Diethylbutylmalonat, Diethylenglycoldibutylether, Diethylethylmalonat, Diethylphenylmalonat, Diethylphthalat, Diethylsebaceat, Diethylsuberat, Diethylsuccinat, Dibutylmaleinat, Dibutylphthalat, Lecithin, Paraffinöl, Petrolatum, flüssige Paraffine, Ester der Sebacinsäure, insbesondere Sebacinsäuredibutylester, Sebacinsäurediethylester, Sebacinsäurediisoprpylester, Sebacinsäuredioctylester. Unter den oben genannten hydrophoben organischen Flüssigkeiten sind weiterhin ganz besonders bevorzugt die dünnflüssigen kurz- und mittelkettigen Triglyceride und mittelkettigen Fettsäureester des Propylenglycols, allein und in Kombination untereinander und mit anderen Substanzen. Insbesondere ganz besonders bevorzugt sind für die medizinische Anwendung von den oben genannten Substanzen und deren Derivaten und Homologen solche, die CFR 21 (Code of Federal Regulations) entsprechen und als "GRAS" (generally recognised as safe) klassifiziert sind.

Für die Verwendung als Implantatmaterialien und Wirkstoffträger sind für die organische Flüssigkeit besonders neutrale Öle mit relativ niedriger Viskosität interessant. Hierbei handelt es sich typischerweise um Ester des Glycerins oder Propylenglycols mit relativ kurzkettigen Fettsäuren. Aus dieser Gruppe sind sowohl die Mono-, Di- als auch Triglyceride interessant. Während die Triglyceride praktisch nicht mit Wasser mischbar sind, können Mono- und Diglyceride je nach Zusammensetzung mit Wasser leicht Emulsionen oder micelläre Lösungen bilden. Diese Aggregate, die den Eindruck molekularer Lösungen machen können, widersprechen nicht der Einschränkung, wonach die Trägerflüssigkeit im chemischen Sinn nicht mit Wasser mischbar ist, da es sich auch hierbei um Mischungen im physikalischen Sinn mit der Ausbildung von Phasengrenzen handelt. Außer Fettsäureestern des Glycerins kommen hier auch die Fettsäureester anderer ein- und mehrwertiger Alkohole in Betracht, insbesondere die des Ethylenglykols und des Propylenglycols (Propandiols) und weiterhin besonders bevorzugt die Fettsäureester der flüssigen Oligomere und/oder Cooligomere aus Ethylenglykol und Propylenglykol. Weitere bevorzugte Ester mehrwertiger Alkohole sind die der Zucker und Zuckeralkohole, wie insbesondere die des Sorbits, Sorbitans und Xylits. Grund für die Präferenz solcher Ester ist die einfache biologische Abbaubarkeit oder enzymatische Spaltbarkeit mit nachfolgender Ausscheidung oder Metabolisierbarkeit der Spaltprodukte. Weiterer Grund ist die einfache synthetische Zugänglichkeit dieser Substanzen, so dass eine große Variabilität an Flüssigkeiten zur Verfügung steht, bzw. verfügbar gemacht werden kann und so den jeweiligen Anforderungen angepasst werden kann. Bevorzugt werden daher allgemein Pasten, Suspensionen oder Dispersionen zur Herstellung von Implantatmaterialien, bei denen die organische Flüssigkeit vorwiegend, also zu > 50% aus Estern von ein- und/oder mehrwertigen Alkoholen mit Fettsäuren besteht.

Eine besondere Form der erfindungsgemäßen Pasten enthält als Trägerflüssigkeit flüssige Oligomere der Hydroxysäuren, die auch zur Synthese resorbierbarer Polymere verwendet werden. Am weitesten verbreitet sind Polymere der Milchsäure, Glykolsäure, Hydroxybuttersäure, des Caprolactons und deren Copolymere. Verbindungen dieser Hydroxysäuren mit niedrigem Polymerisationsgrad sind bei Normalbedingungen viskose Flüssigkeiten. Als besonders geeignete Trägerflüssigkeiten aus dieser Gruppe werden die Oligoester der genannten Hydroxysäuren mit ein- oder mehrwertigen Alkoholen angesehen. Diese Verbindungen sind synthetisch sehr leicht zugänglich und können durch das Verhältnis von Alkohol zu Hxdroxysäuren und das Verhältnis der Mischung der Hydroxysäuren untereinander in ihren Eigenschaften in weiten Bereichen maßgeschneidert werden. Beispiele sind Ethylenglykol-Oligolactid oder Glycerin-Oligolactid, die bei einem molaren Verhältnis von Alkohol zu Milchsäure von > 0,1 fließfähige viskose Flüssigkeiten darstellen, die bei Wasserabschluss lagerstabil und mit Wasser nicht mischbar sind. Vorteilhaft ist bei diesen Trägerflüssigkeiten die leichte biologische Abbaubarkeit und die gute Verträglichkeit mit dem Knochen, die beispielsweise zur Nutzung dieser Oligomere für die Verwendung resorbierbarer Knochenwachse geführt hat (DE 19858891; 19981219). Hier nicht weiter spezifizierte Versuche haben ergeben, dass sich in diese viskosen Flüssigkeiten problemlos große Mengen an mineralischen Feststoffen (wie oben beschrieben) einarbeiten lassen. Bevorzugt werden daher Pasten, Suspensionen oder Dispersionen zur Herstellung von Implantatmaterialien, bei denen die organische Flüssigkeit zu > 50% aus Estern oder Oligo-Estern von ein- oder mehrwertigen Alkoholen mit Hydroxysäuren, insbesondere der Milchsäure, Glykolsäure, Hydroxybuttersäure, Caprolacton, deren Mischungen, CoOligomeren und oder Stereoisomeren besteht.

Für Pasten, Suspensionen oder Dispersionen zur Herstellung von Implantatmaterialien sind weiterhin die flüssigen oligomeren und polymeren Derivate des Ethylenglykols (EO) und des Propylenglycols (PO) besonders geeignet, weil auch die Vertreter dieser Substanzgruppe als biologisch besonders verträglich bekannt sind und sehr vielseitig als Hilfsstoffe in Arzneimitteln, Medizinprodukten und Kosmetika eingesetzt werden (Pluronics und Tetronics).

Als zugehörig zu dieser Gruppe werden auch alle Verbindungen betrachtet, die außer EO - und/oder PO-Einheiten weiter Bausteine zu weniger als 50% (Masse) enthalten. Folglich umfasst diese Gruppe neben PEG (Polyethylenglykol) und PPG (Polypropylenglycol) besonders bevorzugt Copolymere/Cooligomere dieser Bausteine, die sowohl eine zufällige Abfolge der Einheiten haben können, als auch als Block-Copolymere/Cooligomere vorliegen können. Ebenfalls besonders bevorzugt sind (Co-)Polymere/(Co-)Oligomere, die weitere Einheiten aus folgenden Verbindungen enthalten: Alkohole, Fettsäuren, Zuckern, Zuckeralkoholen, Aminen. Typische Vertreter dieser Gruppe sind neben PEG und PPG, deren Copolymere, Alkoholethoxylate, Fettsäureethoxylate, Fettaminethoxylate, Zuckerethoxylate, Ethoxylate der Fettsäureester mehrwertiger Alkohole und Zucker, wie insbesondere die Ethoxylate der Fettsäureester des Sorbits/Sorbitans und hier insbesondere die Vertreter vom Typ der Tween und Span Produkte (Brij, Triton). Allgemein werden in diesem Zusammenhang Pasten, Suspensionen oder Dispersionen zur Herstellung von Implantatmaterialien bevorzugt, bei denen die organische Flüssigkeit zu > 0,1% aus Oligomeren bzw. Polymeren und/oder Cooligomeren bzw. Copolymeren des Ethylenglycols und/oder Propylenglycols miteinander und anderen Bausteinen besteht, wobei die Ethylenglycol- und Propylenglycoleinheiten zusammen mehr als 50% (Masse) der entsprechenden Verbindung ausmachen und wobei die Verbindungen bei Normalbedingungen flüssig sind. Die oben genannten Substanzen und Substanzgruppen sind zumindest teilweise wasserlöslich. In diesen Fällen sind sie als Bestandteile der Trägerflüssigkeit gemeinsam mit nicht wasserlöslichen Substanzen bevorzugt. Die nicht wasserlöslichen Vertreter können auch allein oder in Kombination mit weiteren Substanzen als Hauptbestandteil der Trägerflüssigkeit verwendet werden.

Die Einsatzmöglichkeiten der erfindungsgemäßen Implantatmaterialien sind sehr vielfältig und reichen bei den medizinischen Anwendungen von der Verwendung als implantierbarer Wirkstoffträger, der nur einer geringen mechanischen Belastung ausgesetzt wird, bis zum Knochenzement für die Fixierung von Implantaten, Augmentation von mechanisch hoch belasteten Knochen und Rekonstruktion von Skelettstrukturen, die in vielen Fällen hohen Belastungen ausgesetzt sein können. Der große Vorteil der erfindungsgemäßen Implantatmaterialien ist die Möglichkeit der Anpassung der Eigenschaften an die jeweiligen Einsatzgebiete. Ein wichtiges Stellglied ist dabei die Menge an organomineralischen Feststoffen, die in der Paste dispergiert ist. Bevorzugt muss sie so hoch sein, dass eine Abbindereaktion zwischen den Partikeln der Feststoffdispersion gewährleistet werden kann, wenn diese mit der wässrigen Lösung gemischt wird. Die kritische Konzentration an (reaktiven) Feststoffpartikeln hängt stark von einer Reihe von Faktoren ab. Neben der Art der reaktiven Feststoffe spielen hier vor allem die Partikelgröße und -form eine entscheidende Rolle. In einer ausgewählten Ausführungsform der Erfindung enthalten die Pasten, Suspensionen oder Dispersionen zur Herstellung von Implantatmaterialien die dispergierten mineralischen Feststoffe zu 25% bis 75% (Gewichtsprozent) in der aus organischer Flüssigkeit und Feststoffen bestehenden Paste. In dieser Ausführungsform liegen die Feststoffpartikel zumindest teilweise in vergleichsweise hochdisperser Form vor, wie dies beispielsweise für nanodimensionierte Calciumphosphate, Calciumcarbonate, Calciumhydroxyde, Calciumoxide, Calciumsulfate, Calciumcitrate, Magnesiumoxide, Magnesiumhydroxyd-Carbonate, Silikate verschiedener Kationen und durch Sol/Gel-Verfahren hergestellte reaktive Gläser (wie sie für Glas-Ionomer-Zemente verwendet werden) gegeben sein kann. Anwendungsgebiet sind hier vor allem Wirkstoffträger und Knochenfüllstoffe mit einer sehr hohen biologischen Aktivität.

Nachfolgend sind beispielhaft Zusammensetzungen für die Anwendung als Wirkstoffträger oder Knochenfüllmaterial mit geringer mechanischer Belastung (Gewichtsprozent) aufgeführt:

| Reaktive mineralische Pulver(mischung) | Trägerflüssigkeit | Tenside (s.u.) | Mineralische Füllstoffe | Wirkstoffe |
|---|---|---|---|---|
| 25-75 | 20-50 | 0,1-15 | 0-50 | 0-25 |
| *) 50-70 | 20-35 | 0,5-10 | 0-20 | 0-10 |

| | | | | |
|---|---|---|---|---|
| *) Pasten, Suspensionen oder Dispersionen zur Herstellung von Implantatmaterialien mit einer reaktiven mineralischen Pulvermischung mit einem Anteil von 50 bis 70 Gew.% sind dabei bevorzugt. | | | | |

Die aufgeführten Zusammensetzungen sind auch für 2-Komponentensysteme geeignet, wobei die in der Tabelle aufgeführten Zusammensetzungen für die wasserfreie Komponente gelten.

In einer weiteren Ausführungsform liegt das Augenmerk auf einem besonders hohen Feststoffgehalt der erfindungsgemäßen Paste mit dem Ziel, Zemente mit einer hohen Festigkeit zu erzielen. Dies trifft für praktisch alle technischen Anwendungen zu und im medizinischen Bereich insbesondere für die Verwendung als Knochenzemente und Knochenfüllstoffe für hohe Belastungen. Gegenstand dieser Ausführungsform sind Pasten, Suspensionen oder Dispersionen zur Herstellung von Implantatmaterialien, bei denen die dispergierten organomineralischen Feststoffe zu > 75% (Gewichtsprozent) in der aus organischer Flüssigkeit und Feststoffen bestehenden Paste enthalten sind. Beispiele dieser Ausführungsform sind in den Ausführungsbeispielen genannt. Besonders hohe Gehalte an Feststoffen können erzielt werden, wenn die reaktiven Feststoffe als Pulver mit der Trägerflüssigkeit kombiniert werden und anschließend unter hohem Energieeintrag vermischt und weiter zerkleinert werden, so wie im Beispiel dargestellt. Auf diese Weise lassen sich mit vorwiegend aus Calciumphosphaten bestehenden Feststoffgemischen Füllungsgrade von > 80% erreichen. Eine weitere Erhöhung des Feststoffgehalts lässt sich durch die Zumischung weiterer - typischerweise relativ grober Feststoffe - in die vorgemischte Paste erzielen, so dass bei Mischung einer Paste aus Öl und Calciumphosphaten, die bereits einen Feststoffgehalt von 70% hat, durch Zumischung gesinterter, weitgehend dichter Partikel aus β-TCP im Verhältnis 1:1 eine Paste mit einem Feststoffgehalt von > 85% erhalten werden kann. Dieses Vorgehen ist auch auf andere Stoffsysteme zu übertragen und stellt eine besonders bevorzugte Ausführungsform der Erfindung dar. Bevorzugt werden daher Pasten, Suspensionen oder Dispersionen zur Herstellung von Implantatmaterialien, bei denen die dispergierten organomineralischen Feststoffe zu > 70% (Gewichtsprozent) in der aus organischer Flüssigkeit und Feststoffen bestehenden Paste enthalten sind.

Nachfolgend ist beispielhaft eine Zusammensetzung für die Anwendung als Knochenfüllstoff, als Wirkstoffträger oder Knochenzement mit hoher mechanischer Belastung (Gewichtsprozent) aufgeführt:

| Reaktive mineralische Pulver(mischung) | Trägerflüssigkeit | Tenside (s.u.) | Mineralische oder organische Füllstoffe | Wirkstoffe |
|---|---|---|---|---|
| 75-85 | 10-25 | 0-5 | 0-5 | 0-5 |

Die aufgeführten Zusammensetzungen sind auch für 2-Komponentensysteme geeignet, wobei die in der Tabelle aufgeführten Zusammensetzungen für die wasserfreie Komponente gelten.

Die Verwendung von Substanzen, die bei der Vermischung von Pulvern mit Flüssigkeiten und von Flüssigkeiten untereinander bzw. mit Pasten unterstützend wirken, haben in der vorliegenden Erfindung eine herausragende Bedeutung. Ein Teil der geeigneten Tenside oder Stoffe, die neben anderen Wirkungen auch als Tenside wirken können, liegt in flüssiger Form vor und kann wie oben ausgeführt auch als Teil der Trägerflüssigkeit oder als Trägerflüssigkeit selbst verwendet werden. Hier sei aber ausdrücklich erwähnt, dass auch Tenside, die bei Normalbedingungen als Feststoffe vorliegen, vorteilhaft in den erfindungsgemäßen Pasten, Suspensionen oder Dispersionen zur Herstellung von Implantatmaterialien verwendet werden können, wie sich an Beispiel 2 ebenfalls zeigt (auch Amphisol A und SDS liegen bei Raumtemperatur als Feststoffe vor). Eigene Versuche haben gezeigt, dass der Zusatz von Tensiden die Einmischung der Feststoffe in die Trägerflüssigkeit stark begünstigt und insbesondere die Vermischung der erhaltenen Paste mit einer wässrigen Lösung entscheidend beeinflussen kann. Auf eine umfassende Nennung der geeigneten Tenside sei an dieser Stelle verzichtet, da eine solche Darstellung in keiner Weise vollständig sein kann und es in Anbetracht der vielfältigen Zusammensetzungen der erfindungsgemäßen Implantatmaterialien und der zahlreichen potenziellen Anwendungsfelder praktisch kein Tensid gibt, dass aus objektiven Gründen auszuschließen wäre. Zum Hintergrund und Überblick sei nochmals auf die Monographie "Die Tenside" verwiesen. Bevorzugt werden daher alle Pasten, Suspensionen oder Dispersionen zur Herstellung von Implantatmaterialien, bei denen die Paste außer suspendierten/dispergierten organomineralischen Feststoffen und organischer Flüssigkeit ein oder mehrere Tenside enthält. Nachfolgend seien einige bevorzugte Gruppen von Tensiden speziell hervorgehoben, insbesondere solche, die für die medizintechnische/pharma-zeutische Anwendung in Betracht kommen.
• Anionische Tenside
   o Fettsäuren und deren Salze (Na, K, NH4, Ca, Mg, Zn, Fe)
      ■ Z. B. Natriumoleat, Natriumpalmitat
   o Ester von Fettsäuren und deren Salze
      ■ Z. B. Natriumdilaureth-7-citrat, Stearoyldinatriumtartrat
   o Carbonsäureether
      ■ Z. B. Fettalkoholpolyglykolethercarbonsäure (und Salze)
   o Alkylsulfate/Alkylethersulfate (und deren Salze)
      ■ Z. B. Natriumalkysulfate (bes. Natriumlaurylsulfat)
   o Alkylsulfonate
      ■ Z. B. Natriumlaurylsulfonat
   o Sulfosuccinate
      ■ Z. B. Natriundialkylsulfosuccinat
   o Phosphosäureester (Alkyl- und Alkyletherphosphate) und ihre Salze
      ■ Mono- und Di-Alkylphosphorsäureester
   o Acylaminosäuren und ihre Salze
   ■ Acylglutamate, Acylpeptide, Acylsarkoside
• Kationische Tenside
   o Alkylaminsalze
   o Alkylimidazolinje
   o Tetraalkyl(aryl-) Ammoniumsalze
   o Heterozyklische Ammoniumsalze
      ■ Z. B. Alkylethylmorpholinethosulfat
   o Ethoxylierte Amine
      ■ Z. B. Polyethylenglykol-6-Laurylamin
• Amphothere Tenside
   o Acylethyldiamine und Derivate
   o N-Alkylaminosäuren und/oder Imino-Dicarbonsäuren
   o Betaine
      ■ Z. B. Alkylamidopropylbetain
   o Lecithine
• Nichtionische Tenside
   o Fettalkohole
      ■ z. B. Decylalkohol, Dodecylalkohol (besonders bevorzugt als Cotenside)
   o Ethoxylierte Fettalkohole
      ■ CH3(CH2)x-O-(CH2CH2O)y-H mit x=8-18 und y=ca. 2- ca. 300
   o Ethylenoxid/Propylenoxid-Blockcopolymere
   o Alkylphenolethoxylate
   o Alkylpolyglucoside
   o Ethoxylierte Fette und Öle
   o Alkanolamide
   o Ethoxylierte Alkanolamide
   o Polyehtylenglykolfettsäureester
   o Glykol- und Glykolester
      ■ Ethylenglykolfettsäureester
      ■ Propylenglykolfettsäureester
      ■ Glycerinfettsäureester (Mono- und Diester)
   o Sorbitanester (Mono- und Triester)
   o Zuckerester
   o Ester/Ether-Tenside
      ■ Ethoxylierte Glykol- und Glycerinester
      ■ Ethoxylierte Sorbitanester
         • Ethoxylierte Sorbitanester der Fettsäuren Laurin-, Myristin-, Palmitin-, Stearin- und Ölsäure (z. B. Tween-Typen)
      ■ Polyglycerinmonoester
      ■ Aminoxide
• Alkoxylierte Polysiloxane (Silicontenside)
• Flourtenside

Die beiden letztgenannten Gruppen haben vor allem technisches Anwendungspotenzial in Kombination mit den erfindungsgemäßen Pasten, Suspensionen oder Dispersionen zur Herstellung von Implantatmaterialien.

Nachfolgend sollen daher nur einige ausgewählte Tenside genannt werden, deren Verwendung in den erfindungsgemäßen Pasten, Suspensionen oder Dispersionen zur Herstellung von Implantatmaterialien bevorzugt ist (andere Tenside, Derivate oder Homologe der genannten Tenside sollen damit aber in keiner Weise ausgeschlossen sein): Natriumlaurylsulfat, Glycerinmonooleat, Polysorbat 20, 21, 40, 60, 61, 65, 80, 81, 85, 120, Sorbitandiisostearat, Sorbitandioleat, Sorbitanmonoisostearat, Sorbitanmonolaurat, Sorbitanmonololeat, Sorbitanmonopalmitat, Sorbitanmonostearat, Sorbitantrioleat, Sorbitantrilaurat, Sorbitantricaprylat/-caprat, Isopropylmyristat, Lecithin, Lysolecithine, Oleinsäure, Ölsäure, Polyethylenglycolmonocetylether, Polyethylenglycolmonostearylether, Polyethylenglycolmonolaurylether, Polyethylenglycolmonooleylether, polyethoxyliertes Rizinusöl, Polyoxyl-40-stearat, Polyoxyl-50-stearat, Ascorbylpalmitat, Cetylphosphat ((Amphisol®)und dessen Salze), PEG-Cetyl-Stearyl Ether (Ceteareth-6 (-11, -25) Cetyl-/Stearylalkohol (Cremophor A®-Typen).

Ganz besonders bevorzugt für die medizintechnische und pharmazeutische Anwendung der vorliegenden Erfindung sind Tenside, die bereits in pharmazeutischen Präparationen eingesetzt werden. Besonders hervorgehoben sind hier die unter den Namen Tween 20, Tween 80, Pluronic F68 und Cremophor angebotenen Produkte und das Lecithin. Weiterhin ganz besonders bevorzugt sind die Fettsäuren und ihre Salze und die mittelkettigen Fettalkohole.

Außer Tensiden können auch feinst verteilte Feststoffe die Ausbildung und Stabilität von physikalischen Mischungen zwischen Feststoffen und Flüssigkeiten und Flüssigkeiten untereinander unterstützen (siehe die Arbeit "Makro- und Mikroemulsionen" von H.-D. Dörfler: Grenzflächen und kolloid-disperse Systeme. Springer, Berlin, 2002, Kap. 13.). Bevorzugt sind daher insbesondere auch alle Ausführungsformen der erfindungsgemäßen Pasten, Suspensionen oder Dispersionen zur Herstellung von Implantatmaterialien, bei denen die Paste außer suspendierten/dispergierten organomineralischen Feststoffen, die an der zementartigen Abbindereaktion beteiligt sind, organischer Flüssigkeit und ggf. weiteren Hilfs- und Wirkstoffen Eisenoxide, Tonmineralien, Siliciumdioxide, Bariumsulfat oder Glycerinstearat als hochdisperse Feststoffe enthalten, die eine Ausbildung und die Stabilität physikalischer Mischungen (Emulsionen) unterstützen. Die obige Aufzählung erhebt keinen Anspruch auf Vollständigkeit und soll daher auch andere Substanzen umfassen, die in gleicher Weise wirken.

Unter den weiterhin sinnvollen und/oder notwendigen Hilfsstoffen zur optimalen Ausgestaltung erfindungsgemäßer Produkte kommt den Polymeren eine besondere Bedeutung zu. Über sie kann insbesondere die Viskosität der Paste und ihre Lagerstabilität beeinflusst werden. Weiterhin haben sie Einfluss auf die Kohäsion der Paste, was insbesondere in der Ausführungsform als Ein-Komponenten-System von großer Bedeutung sein kann. In medizinischen Anwendungen können sie die biologische Aktivität erhöhen oder die Resorbierbarkeit beeinflussen. Als Polymere kommen insbesondere die Biopolymere Kollagen und dessen Derivate (u. a. auch Gelatine), Stärke und deren Derivate (insbesondere Hydroxyethylstärke und Carboxymethylstärke), Cellulosederivate, Chitin/Chitosan-Derivate und die synthetischen Polymere Polyvinylalkohol, Polyvinylpyrrolidon, Polyacrylsäure, Polymethacrylsäure, sonstige Polyacrylderivate (z. B. unter dem Warenzeichen Eudragit bekannte Polyacrylsäure Derivate der Firma Degussa), hochmolekulare PEG und PPG Derivate (insbesondere auch hochmolekulare Pluronics und Tetronics und homologe Polyether) in Betracht. Die obige Aufzählung erhebt keinen Anspruch auf Vollständigkeit und soll daher auch andere Substanzen umfassen, die in ähnlicher Weise wirken. Bevorzugt werden allgemein Pasten, Suspensionen oder Dispersionen zur Herstellung von Implantatmaterialien, bei denen die Paste außer suspendierten/dispergierten organomineralischen Feststoffen und organischer Flüssigkeit und ein oder mehreren Tensiden und/oder hochdispersen Feststoffen gelöste oder suspendierte Polymere mit einem Molekulargewicht von > 2500 enthält.

Besonders hervorzuheben sind an dieser Stelle Polymere, hochdisperse Feststoffe und Tenside, die zwar in der Paste vorgelegt werden, die ihre Funktion aber während und nach der Vermischung mit einer wässrigen Lösung vor allem durch die Beeinflussung der Eigenschaften der wässrigen Lösung ausüben sollen. Das bezieht sich insbesondere auf die Bildung der Emulsion aus organischer Flüssigkeit und wässriger Lösung über die Beeinflussung der Viskosität und der Oberflächenspannung.

Die erfindungsgemäßen Pasten, Suspensionen oder Dispersionen zur Herstellung von Implantatmaterialien sind geradezu prädestiniert für die Verwendung als Wirkstoffträger im Knochen- und Zahnbereich, insbesondere für Wirkstoffe, die lokal wirksam sind. Die Möglichkeit, solche Wirkstoffe entweder in der organisch basierten Paste oder in der wässrigen Lösung vorlegen zu können, macht diese Materialien praktisch für alle relevanten Wirkstoffklassen zum geeigneten Trägermaterial. Zudem kann die Porosität des abgebundenen Feststoffs in weiten Bereichen eingestellt werden, um so auch die Freisetzungskinetik den Anforderungen anpassen zu können. Die Verwendung geeigneter Hilfsstoffe, wie insbesondere die oben genannten hochdispersen Feststoffe, Tenside und Polymere, lässt zudem eine weitere Feineinstellung der Wirkstofffreisetzung zu. Als geeignete Wirkstoffe kommen alle für die lokale Behandlung von Knochenerkrankungen relevanten Substanzen in Betracht, insbesondere solche mit einer antimikrobiellen Wirkung (wie z. B. Antibiotika, Antiseptika, antimikrobielle Peptide), einer Antitumorwirkung (wie z. B. Cytostatika, Antikörper, Hormone), antiresorptiven Wirkung (wie z. B. Bisphosphonate, Cortikoide, Fluor, Protonenpumpen-Inhibitoren), antiinflammatorischen Wirkung, knochenwachstumsstimulierenden Wirkung (Wachstumsfaktoren, Vitamine, Hormone, Morphogene), etc. Auch hier kann die obige Aufzählung keinen Anspruch auf Vollständigkeit erheben und soll daher auch andere Substanzen umfassen, die lokal im Knochen und den angrenzenden Geweben wirksam sein können und die Funktion des Implantatmaterials in gewünschter Weise unterstützen können. Insgesamt sind also alle Pasten, Suspensionen oder Dispersionen zur Herstellung von Implantatmaterialien bevorzugt, bei denen die Paste außer dispergierten organomineralischen Feststoffen und organischer Flüssigkeit einen oder mehrere gelöste oder suspendierte pharmakologische Wirkstoffe enthält.

Vorzugsweise enthalten die Implantatmaterialien außer der Paste/Suspension oder Dispersion als weitere Komponente eine wässrige Lösung oder reines Wasser. In dieser Ausführungsform wird das Implantmaterial so ausgelegt, dass die nach einer der obigen Spezifizierungen hergestellte Paste aus organischer Flüssigkeit und dispergierten Feststoffen mit einer definierten wässrigen Lösung oder ggf. reinem Wasser (eine besondere Form der definierten Lösung) gemischt wird und diese Mischung anschließend zu einem Feststoff aushärtet.

Diese Ausführungsform hat gegenüber der weiter unten genannten zweiten Ausführungsform den Vorteil, dass die wässrige Lösung so eingestellt werden kann, dass sowohl die Aushärtung, als auch weitere Eigenschaften des Endprodukts gezielt beeinflusst werden können. Dazu können der wässrigen Lösung Substanzen zugesetzt werden, die selbst auch an der Abbindereaktion teilnehmen oder deren Kinetik beeinflussen. Weiterhin können der wässrigen Lösung insbesondere Substanzen zugesetzt werden, die die Vermischung der wässrigen Lösung mit der Paste in einer gewünschten Weise beeinflussen. Grundsätzlich gehören dazu alle nicht reaktiven Bestandteile von mineralischen Zementen, insbesondere Füll- und Wirkstoffe, die auch in der wässrigen Lösung formuliert sein können. Sie können dort sowohl gelöst, als auch dispergiert oder suspendiert vorliegen. Die wässrige Lösung kann dementsprechend ebenfalls den Charakter einer Paste annehmen, die einen erheblichen Feststoffanteil enthält. Da die homogene Vermischung von wasserfreier Paste mit der wässrigen Lösung in 2-Komponentensystemen bei möglichst ähnlicher Viskosität und Konsistenz der beiden Komponenten am besten gelingt, ist die Formulierung der wässrigen Lösung mit suspendierten Füllstoffen, hochdispersen Feststoffen und/oder gelösten oder gequollenen Polymeren eine bevorzugte Ausgestaltungsform der Erfindung. Beispiele für geeignete Füllstoffe sind CaCO3, feinteilige Calciumphosphate (insbesondere nanokristallines Hydroxylapatit), feinteilige Magnesiumphosphate oder -carbonate, hochdisperse Kieselsäuren, bzw. deren Salze und kolloidal gelöste Polymere.

Besonders zu erwähnen sind Zusätze zur wässrigen Lösung, über die deren Viskosität beeinflusst werden kann, also insbesondere Zusätze von Polymeren, hochdispersen Feststoffen und Tensiden, von denen einige (wie insbesondere die hochethoxylierten Sorbitanester und Pluronics [PEG-PPG Block-Copolymere]) mit wässrigen Lösungen in bestimmten Konzentrationen viskose Gele bilden, die für die erfindungsgemäßen Implantatmaterialien von Bedeutung sind.

Nach einer Ausführungsform der Erfindung sind nichtreaktive Bestandteile der mineralischer Zementfeststoffe teilweise oder vollständig in der wässrigen Lösung enthalten.

Speziell erwähnt werden sollen auch Substanzen als Zusatz zur wässrigen Lösung, die zwar nicht selbst an der Abbindereaktion der mineralischen Feststoffe teilnehmen, aber beispielsweise deren Kristallstruktur beeinflussen können und so die Eigenschaften der sich bildenden Endprodukte entscheidend modifizieren können. Das können polymere Substanzen wie Hyaluronsäure, Chondroitinsulfat oder Polyacrylate sein, niedermolekulare organische Stoffe wie Zitronensäure, Weinsäure, Aminosäuren, Zucker und deren Derivate und deren jeweilige Salze oder anorganische Substanzen sein, wobei letztere in der Regel in die Kristallstruktur zumindest teilweise eingebunden werden.

Relevante Wirkstoffe können auch in der wässrigen Lösung gelöst oder suspendiert vorgelegt werden, sofern sie in dieser Form ausreichend stabil sind. Die chemische Stabilität und die Freisetzungskinetik bestimmen dabei weitgehend, ob ein Wirkstoff bevorzugt in der öligen oder der wässrigen Phase vorgelegt wird.

Grundsätzlich können die Implantatmaterialien, die aus zwei oder mehr Komponenten bestehen, auf verschiedene Arten und Weisen miteinander kombiniert und gemischt werden, um so eine Abbindereaktion auszulösen und die Bildung eines Feststoffs zu erreichen. Besonders bevorzugt ist dabei allerdings, dass die jeweilige Komponente in Form einer Paste oder (viskosen) Flüssigkeit in einem Behältnis vorgelegt wird und die Komponenten des Implantatmaterials vor oder während der Anwendung so miteinander und mit einer Mischvorrichtung kombiniert werden, dass sie über diese Mischvorrichtung makroskopisch homogen vermischt werden können. Im einfachsten Fall werden dazu 2 Pasten, von denen eine die ölige Phase bildet und die zweite die wässrige Phase, jeweils in getrennten Kartuschen abgefüllt. Unmittelbar vor der Anwendung werden die Kartuschen über eine Öffnung an ihrem einen Ende über ein T-Stück miteinander und mit einem Statikmischer verbunden. Die beiden Kartuschen werden mittels einer Vorrichtung, die eine gleichmäßige Austragung beider Kartuschen gewährleistet, ausgetragen. Dabei werden die beiden Pasten in den Statikmischer gedrückt und beim Durchströmen makroskopisch homogen miteinander gemischt. Derartige Vorrichtungen sind im Prinzip bekannt und werden beispielsweise für die Mischung von Fibrinogen und Thrombin bei der Verwendung autologer Fibrinkleber genutzt. Im Rahmen dieser Erfindung werden sie erstmals für die Mischung der erfindungsgemäßen Implantatmaterialien beschrieben.

Beispielhafte Zusammensetzungen von 2 Komponentensystemen für die Anwendung als Knochenfüllstoff oder Knochenzement (Angaben in Gewichtsprozent bezogen auf den Gesamtgehalt der jeweiligen Komponente):

**Wasserfreie Paste (Paste1)**

| Reaktive mineralische Pulver(mischung) | Trägerflüssigkeit (Öl) | Tenside | Mineralische oder organische Füllstoffe | Wirkstoffe |
|---|---|---|---|---|
| *)25-75 | 10-49 | 0,1-15 | 0-50 | 0-25 |
| **)70-89 | 10-29 | 0,1-15 | 0-19 | 0-19 |

| | | | | |
|---|---|---|---|---|
| *) bevorzugte Verwendung als Wirkstoffträger und gering belasteter Füllstoff **) bevorzugte Verwendung als Knochenfüllstoff, Wirkstoffträger oder Knochenzement mit hoher Belastbarkeit | | | | |

**Wässrige Lösung (Paste2)**

| Wasser | Wasserlösliches Polymer | Tenside | Mineralische oder organische Füllstoffe / Abbindebeschleuniger | Wirkstoffe |
|---|---|---|---|---|
| 25-100 | 0-30 | 0-15 | 0-70 | 0-25 |

Beide Komponenten können in weiten Bereichen miteinander gemischt werden. Bevorzugt sind Mischungsverhältnisse im Bereich zwischen 10:1 und 1:1 (Paste1 : Paste2); Abbindebeschleuniger sind in der wässrigen Lösung gelöste oder suspendierte Stoffe (z. B. Phosphatsalze oder organische Säuren, bzw. deren Salze), die die Abbindekinetik des mineralischen Zements beeinflussen.

In einer besonders bevorzugten Ausführungsform eines Mehrkomponenten-Applikationssystems mit einer Mischvorrichtung werden die beiden Pasten in jeweils eine Kammer einer handelsüblichen Doppelkammerspritze abgefüllt und verschlossen. Im Falle einer invasiven medizinischen Anwendung werden sie in dieser Form sterilisiert. Bei der Anwendung wird die Doppelkammerspritze mit einem Statikmischer verbunden und entweder manuell, mittels eines übersetzten Handgeräts (Pistole) oder über eine fremdangetriebene Vorrichtung ausgetragen und gemischt. Bei dieser Vermischung der beiden Komponenten wird die Abbindereaktion in Gang gesetzt. Auch die entsprechenden Doppelkammerspritzen und Statikmischer sind prinzipiell bekannt (z. B. als Produkte der Fa. Mixpac), werden hier aber erstmals für die Applikation der erfindungsgemäßen Implantatmaterialien beschrieben.

In einer weiteren, ebenfalls besonders bevorzugten Ausführungsform der Erfindung als Einkomponenten Applikationssystem besteht das Implantatmaterial nur aus einer Komponente, die alle zur Auslösung einer Abbindereaktion notwendigen Substanzen außer Wasser enthält (ggf. enthaltend weitere Hilfs- und Wirkstoffe) und in der bei Einbringung in ein wässriges Medium und/oder Kontakt mit wasserhaltigen Oberflächen spontan einer Abbindereaktion ausgelöst wird. Typischerweise enthält das Implantatmaterial in dieser Ausführungsform (organo) mineralische Pulver, Tensid(e) und ggf. weitere Hilfs- und Wirkstoffe kombiniert in einer Paste. Diese Paste kann mit Wasser analog der vorangegangenen Beschreibungen angemischt werden. Das besondere dieser Ausführungsform besteht allerdings in der Tatsache, dass die Paste, bestehend aus einer Komponente, ohne weitere Anmischung in eine wässrige Umgebung eingebracht werden kann und unter Aufnahme von Wasser aus der Umgebung, bzw. Austausch von organischer Flüssigkeit (Öl) gegen Wasser, zu einem Feststoff abbindet.

Vorteilhafterweise kann das erfindungsgemäße Implantatmaterial in dieser Ausführungsform als einfache Kartusche, z. B. als Spritze ausgebildet sein und somit als sehr einfache und anwendungsfreundliche Ausführungsform fungieren. Besonders geeignet ist diese Ausführungsform für Applikationen der Implantatmaterialien in relativ dünner Schicht, die allseitigen Kontakt zu wässrigen Oberflächen oder Flüssigkeiten hat oder in denen der Werkstoff längere Zeit zur vollständigen Aushärtung hat. Besonders vorteilhaft ist diese Formulierung für die Anwendung als organomineralischer Klebstoff für feuchte Werkstoffoberflächen und insbesondere für die Verklebung von Knochenstrukturen.

Die Implantatmaterialien können für viele technische, medizin(techn)ische und/oder pharmazeutische Anwendungen nutzbringend eingesetzt werden. Die dargestellten Aspekte der Erfindung machen die Implantatmaterialien besonders geeignet für die Herstellung von Medizinprodukten und Arzneimitteln und hier insbesondere für die Herstellung von Knochenzementen, Knochenersatzmaterialien, Knochenklebern, Zahnfüllmaterialien und/oder implantierbaren Wirkstoffträgern. Bevorzugte Ausführungsformen der Erfindung sind Ein- oder Zweikomponentensysteme, die vollständig aus biokompatiblen Materialien bestehen, in sterilisierter Form angeboten werden und im Körper aushärten.

Auf Grund dieser Vorteile ist die Verwendung der erfindungsgemäßen Pasten, Suspensionen oder Dispersionen zur Herstellung von Implantatmaterialien zur Herstellung von Knochenersatzmaterial, Knochenzementen, Knochenklebern und implantierbaren Wirkstoffträgern besonders bevorzugt. Knochenzemente sind entsprechend des Implantatmaterials aufgebaut und an die spezifischen Anwendungen angepasst. Knochenersatzmaterialien sind Zubereitungen aus dem erfindungsgemäßen Implantatmaterial, z. Bsp. zur Auffüllung von Knochendefekten. In diesem Fall kann es vorteilhaft sein, das Knochenersatzmaterial aus der erfindungsgemäßen Paste, Suspension oder Dispersion zur Herstellung eines Implantatmaterials vor der Implantation in den Körper in Form eines ausgehärteten soliden Werkstücks zu formen und anschließend in den Knochendefekt einzubringen/zu implantieren.

Knochenkleber sind Zubereitungen aus dem erfindungsgemäßen Pasten, Suspensionen oder Dispersionen zur Herstellung von Implantatmaterialien, z. B. zur Verbindung und Befestigung von Knochenfragmenten nach Knochenbrüchen oder zur Befestigung von z. B. metallischen oder keramischen oder polymeren Implantatmaterialien am oder im Knochen. In dieser Funktion enthalten die Knochenkleber neben den erfindungsgemäßen Implantatmaterialien adhäsionsfördemde Substanzen, die beispielsweise aus dem Dentalbereich als Adhäsionspromotoren bekannt sind und/oder Substanzen wie Polyacrylate, und/oder aus anionischen Monomeren hergestellte Polymere und/oder Copolymere. Diese Substanzen werden im Dentalbereich u. a. zur Verbesserung der Haftung von Füllungsmaterialien an der Zahnsubstanz verwendet (die mit der Knochensubstanz große Ähnlichkeit hat). Mit den erfindungsgemäßen Pasten, Suspensionen oder Dispersionen zur Herstellung von Implantatmaterialien sind sie besonders vorteilhaft und vielseitig kombinierbar, da sie sowohl mit der Paste, als auch mit der wässrigen Lösung in weiten Konzentrationsbereichen kombiniert werden können. Dies unterscheidet die erfindungsgemäßen Pasten, Suspensionen oder Dispersionen zur Herstellung von Implantatmaterialien von konventionellen Zubereitungen, weil insbesondere die in dieser Hinsicht besonders wirksamen, geladenen oder stark polaren Adhäsionspromotoren sich mit konventionellen mineralischen Zementen nicht ohne weiteres kombinieren lassen. Die Zugabe der Adhäsionspromotoren bewirkt eine stärkere Anhaftung des Implantatmaterials am Knochengewebe.

Wirkstoffträger sind Zubereitungen aus erfindungsgemäßen Pasten, Suspensionen oder Dispersionen zur Herstellung von Implantatmaterialien, die pharmakologische Wirkstoffe in Konzentrationen enthalten, die eine therapeutische Funktion im menschlichen oder tierischen Organismus bewirken können. Diese Wirkstoffe werden nach der Implantation aus dem Implantatmaterial freigesetzt. Bevorzugt ist daher die Kombination mit Wirkstoffen, die eine direkte lokale Wirkung entfalten, die also direkt auf das Gewebe in der Umgebung des Implantatmaterials wirken. Bevorzugt ist die Kombination mit Wirkstoffen, die den Knochenstoffwechsel stimulieren und lokalen Entzündungen entgegenwirken. Besonders bevorzugt sind Wirkstoffträger auf der Basis der erfindungsgemäßen Pasten, Suspensionen oder Dispersionen zur Herstellung von Implantatmaterialien, die antimikrobielle Wirkstoffe wie Antibiotika enthalten, Knochen-anabole Proteine und/oder Peptide wie Bone Morphogenetic Proteins (BMPs) oder von Parathormon abgeleitete Peptide enthalten oder Hemmstoffe der Knochenresorption wie Bisphosphonate oder Protonenpumpeninhibitoren enthalten.

Eine besonders bevorzugte Anwendung der erfindungsgemäßen Pasten, Suspensionen oder Dispersionen zur Herstellung von Implantatmaterialien besteht in der Auffüllung von Knochendefekten und in der Augmentation von osteoporotischem Knochen. In den allermeisten Fällen erfolgt diese Applikation in minimal invasiver Operationstechnik.

Ein typisches Beispiel dafür sind die verschiedenen Verfahren der Vertebroplastie, die in den letzten Jahren immer populärer geworden sind und deren klinische Wirksamkeit immer besser dokumentiert wird. Es ist daher davon auszugehen, dass diese Behandlungstechniken weiter ausgearbeitet werden und die behandelten Fälle stark zunehmen werden. Die Weiterentwicklung der Methoden der Vertebroplastie (inkl. der sogenannten Kyphoplastie) sind ganz wesentlich von der Verfügbarkeit verbesserter Augmentationsmaterialien abhängig. Die bisherigen Knochenzemente sind nur bedingt geeignet, auch wenn einige Produkte speziell für die Vertebroplastie angeboten werden, sind sie doch nach wie vor nur konventionelle Knochenzemente mit geringfügig modifizierter Viskosität, Aushärtekinetik und erhöhtem Gehalt an Röntgenkontrastmaterial. Die erfindungsgemäßen Pasten, Suspensionen oder Dispersionen zur Herstellung von Implantatmaterialien sind prinzipiell in diesen Anwendungen überlegen, weil sie besonders in ihrer Handhabung deutlich einfacher sind. Für die klinisch erfolgreiche Anwendung müssen sie mit geeigneten Applikationssystemen zur Einbringung des Implantatmaterials in den Knochen kombiniert werden. Dazu können im einfachsten Fall einfache Spritzensysteme verwendet werden, wie sie auch heute noch in der Vertebroplastie eingesetzt werden. Zweckmäßiger ist allerdings die Mischung des Implantatmaterials in der Doppelkammerspritze und einem Statikmischer und die Kombination mit einer Injektionskanüle bzw. einem geeigneten Rohr, das bis an die Implantationsstelle reicht. Vorteilhaft ist hier insbesondere, dass das Implantatmaterial erst bei der Extrusion gemischt wird, also in einfacher Weise mit einer Zementkartusche mehrere Injektionen gemacht werden können und es keiner zeitlich abgestimmten Vorarbeit bedarf, weil das Polymersystem gleich anwendungsbereit aus der Spritze kommt. Als Einkomponentensystem kann die Paste, Suspension oder Dispersion zur Herstellung eines Implantatmaterials sogar völlig ohne vorherige Mischung in den Knochen appliziert werden. Im Hinblick auf den in der Einleitung dargestellten Stand der Technik ist dies ein erheblicher anwendungstechnischer Vorteil und macht jegliche Schulung des OP-Personals zur Vorbereitung des Implantatmaterials überflüssig.

Eine besonders bevorzugte Anwendung ist das als Kyphoplastie bekannte Verfahren, bei dem zunächst der osteoporotische Knochen des Wirbelkörpers mit einem Ballon aufgedehnt und in der Umgebung komprimiert wird und anschließend in den erzeugten Hohlraum ein Knochenzement eingebracht wird. Gerade bei dieser Anwendung ist die Kombination des Applikationsinstrumentariums mit den erfindungsgemäßen Pasten, Suspensionen oder Dispersionen zur Herstellung von Implantatmaterialien besonders vorteilhaft, weil in diesem Fall ein vorteilhaftes Applikationssystem mit einem vorteilhaften Füllmaterial kombiniert werden kann. Es werden daher alle Kombinationen der erfindungsgemäßen Pasten, Suspensionen oder Dispersionen zur Herstellung von Implantatmaterialien mit Applikationssystemen bevorzugt, die geeignet sind, das erfindungsgemäße Implantatmaterial in Knochendefekte, Frakturspalten, osteoporotischen Knochen, Knochentumore oder auf andere Art rarifizierte Knochenstrukturen in minimal invasiver Art und Weise einzubringen. Ebenfalls werden alle Zusätze bevorzugt, die an ein Mischsystem vom Typ der genannten Doppelkammerspritzen angebaut werden können oder mit einem solchen System verbunden werden zu dem Zweck, um mit deren Hilfe die erfindungsgemäßen Pasten, Suspensionen oder Dispersionen zur Herstellung von Implantatmaterialien an den Zielort zu applizieren.

Die erfindungsgemäßen Pasten, Suspensionen oder Dispersionen zur Herstellung von Implantatmaterialien lassen sich in besonders vorteilhafter Weise intraoperativ handhaben, also für die Einbringung in den Körper präparieren. Das unterscheidet sie grundsätzlich von konventionellen Knochenzementen, die aus Pulver und Flüssigkeit angemischt werden. Gleichzeitig erlauben die Materialeigenschaften die einfache und zuverlässige minimal invasive Applikation über eine Kanüle. Die freie Kombinierbarkeit der ersten und der zweiten Komponente des Implantatmaterials in einem sehr breiten Mischungsverhältnis (z. B. 1:1, 1:2, 1:4, 1:10) ermöglicht auch die Kombination des Implantatmaterials mit den verschiedensten biologischen Faktoren, die erst am Implantationsort zugesetzt werden können oder sollen. Das gilt besonders für Serumbestandteile wie Blutplättchen-reiches Plasma, intraoperativ gewonnene Zellsuspensionen und durch vorherige Kultivierung gewonnene Suspensionen.

### Ausführungsbeispiele

Anhand nachfolgender Ausführungsbeispiele wird die Erfindung näher erläutert:

### Ausführungsbeispiel 1: Calciumphosphatzement CPC als Zwei-Komponenten-System

### Erste Komponente

Der verwendete CPC besteht aus 60 Gew.-Teilen α-TCP, 26 Gew.-Teilen CaHPO4, 10 Gew.-Teilen CaCO3 und 4 Gew.-Teilen gefälltem Hydroxylapatit. Die vorher zerkleinerten Pulver werden in einer Kugelmühle gemischt und intensiv vermahlen.

100 g der Pulverkomponente des CPC werden mit 20 ml Miglyol 812 in einer Planeten-Kugelmühle (Fritsch Pulverisette 6) in einem 500 ml Becher mit 50 Kugeln (10 mm Durchmesser; jeweils aus Zirkondioxid) für 3x15 min (jeweils 30 min Pause) bei einer Drehzahl von 500 Upm gemischt. Das Ergebnis ist eine homogene viskose Paste mit der Konsistenz von Erdnussbutter.

### 2. Komponente

Als wasserhaltige Trägerflüssigkeit werden 6% Hydroxyethylstärke, 5% Tween 80 und 4% Na₂HPO₄ in Wasser angesetzt.

Mit den beiden Komponenten werden folgende Versuche geführt:
a) Zur Herstellung eines Implantatmaterials werden 10 g der Paste mit 2 ml der wässrigen Lösung in einem Mischbecher vorgelegt und mit einem Spatel gemischt. Paste und wässrige Flüssigkeit lassen sich in kurzer Zeit homogen verreiben, wobei die Viskosität der Mischung langsam ansteigt. Ca. 3 min. nach Beginn der Vermischung zieht die Paste an und erreicht eine Anfangsfestigkeit. Nach 6 Stunden erreicht die erhaltene Zubereitung eine Druckfestigkeit von ca. 12 MPa, nach 3 Tagen liegt die Druckfestigkeit bei 15 MPa.
b) Der Versuch unter a) wird nach einer Lagerung der Paste von 6 Monaten unter Normalbedingungen wiederholt. Die Paste wurde dazu in einem einfachen Schraubdeckelglas ohne weitere Maßnahmen aufbewahrt. Das Mischungsverhalten zeigt keine erkennbaren Unterschiede zum Versuch unter a). Die Druckfestigkeit nach 6 Stunden liegt bei 12 MPa, nach 3 Tagen bei 15 MPa.
c) Der Versuch unter a) wird mit 10 g der Paste und 2,5 ml der wässrigen Lösung wiederholt. Mit der vergrößerten Menge an wässriger Lösung dauert es etwas länger bis Paste und Lösung homogen verteilt sind. Sonst sind keine erkennbaren Unterschiede zwischen beiden Versionen zu verzeichnen. Die Anfangsfestigkeit wird ebenfalls nach 3 min erreicht. Die Druckfestigkeit nach 6 Stunden beträgt 10 MPa, nach 3 Tagen 14 MPa.
d) Der Versuch unter c) wird mit einer konventionellen Doppelkammerspritze der Firma Mixpac (Volumen: 10 ml; Verhältnis der Kartuschen: 4:1) wiederholt. Nach einer geringen Inhomogenität zu Beginn der Austragung ist der weitere ausgetragene Strang homogen und härtet wie die manuell gemischte Probe in ca. 3 min aus.

In Beispiel 1 wurde im Hinblick auf eine Verwendung des Zements in einer Zwei-Kammer-Spritze (Mixpac) eine sehr kurze Abbindezeit gewählt, da in diesem Fall der Zement direkt an den Implantationsort appliziert werden kann. Für eine offene Anmischung im Mischbecher kann die initiale Abbindezeit auf einen Wert von ca. 5-7 Minuten eingestellt werden, indem die Konzentration der Na2HPO4-Lösung auf 1,0% verringert wird. Überraschend und bemerkenswert ist in diesem Zusammenhang auch die Feststellung, dass der Calciumphosphat-Zement nach Beispiel 1 schneller abbindet, als der korrespondierende konventionelle Calciumphosphat-Zement. Die Ergebnisse zeigen damit, dass mit der neuartigen Formulierung Zemente erhalten werden, die zu einem Feststoff reagieren. Die erhaltenen Festigkeitswerte der Feststoffe liegen ca. 30-40% niedriger, als bei den konventionellen Gegenstücken aus Pulver und Anmischflüssigkeit. Allerdings sind bei weiterer Optimierung deutlich höhere Festigkeitswerte zu erwarten (auch der konventionelle Basiszement ist das Ergebnis umfangreicher Optimierungsarbeiten) und zudem liegen die gemessenen Werte auf oder über dem Niveau des spongiösen Knochens und damit sind auch bereits die getesteten Formulierungen für den klinischen Einsatz in zahlreichen Indikationen geeignet, insbesondere bei solchen, bei denen es nicht entscheidend auf die mechanischen Eigenschaften des Knochenersatzmaterials ankommt - was in der Mehrzahl der Fälle auch zutrifft.

### Ausführungsbeispiel 2: Calciumphosphatzement als Ein-Komponenten-System

20 g CPC-Zementpulver nach Beispiel 1 werden mit 4 ml Miglyol 812, 300 mg Na₂HPO₄, 500 mg Tween 80 und 200 mg Amphisoi A manuell vorgemischt und anschließend in einem 100 ml Becher mit 10 Kugeln mit 10 mm Durchmesser (Zirkondioxid-Ausführung) 3x15 min mit jeweils 30 min Pause bei 500 Upm gemischt. Das Ergebnis ist eine homogene viskose Paste. Die Paste wird in eine 10 ml Spritze abgefüllt und anschließend in ein Becherglas mit simulierter Körperflüssigkeit (SBF) (ohne Kanüle) injiziert. Der extrudierte Strang bleibt auch bei leichtem Schütteln völlig intakt und härtet in weniger als 60 min soweit aus, dass er ohne Zerfall aus der Flüssigkeit entnommen werden kann. Die Flüssigkeit bleibt vollkommen klar, woraus erkennbar ist, dass keine Auswaschung von Zementpartikeln erfolgt. Die endgültige Festigkeit wird wie bei konventionell angemischtem Pulver + Flüssigkeit nach ca. 24 Stunden erreicht.

### Ausführungsbeispiel 3, Einkomponenten Zemente

Einkomponenten Zemente gemäß nachfolgender Rezeptur wurden nach Ausführungsbeispiel 2 formuliert und getestet, indem die erhaltenen jeweiligen Pasten in eine 6x6x12 mm Form gegeben wurden und in simulierter Körperflüssigkeit zur Aushärtung gebracht wurden. Die Druckfestigkeit wurde auf einer Universal-Testmaschine nach 100 Stunden Inkubationszeit bestimmt. Die Bestimmung der Abbindezeit erfolgte nach ASTM 266C. Die Konsistenz der Pasten war auf einen Wert vergleichbar mit Erdnussbutter (bei Raumtemperatur) eingestellt.

| Ein-Pasten Zemente | | | | | | | |
|---|---|---|---|---|---|---|---|
| Typ | Zementpulver | Öl | Tween | Amphisol | Beschleuniger/ Reaktant | Druckfestigkeit | Abbindezeit |
| CPC | 80% | 15% | 3% | 1% | 1% Na2HPO4 | 14 MPa | 5 min |
| MgPC *) | 74% | 13% | 0,8% | 2% | 10% (NH4)2HPO4 | 13 MPa | 6 min |

| | | | | | | | |
|---|---|---|---|---|---|---|---|
| CPC: Calciumphosphat-Zement nach Beispiel 1 MgPC: Magnesiumphosphat-Zement; die Herstellung des MgPC-Zementpulvers erfolgte durch Sinterung bei 1100°C über 3 Stunden von MgHPO₄, und Mg(OH)₂ in einem Mengenverhältnis (2:1), das eine molare Zusammensetzung von Mg₃(PO4)₂ ergibt. Nach der Sinterung wurde das Zementpulver in einer Kugelmühle über 4 h auf eine mittlere Partikelgröße von < 25 µm vermahlen. Amphisol: Tensid *) Magnesiumphosphat-Zement bindet durch Reaktion mit Ammoniumsalzen ab, in diesem Beispiel mit di-Ammoniumhydrogenphosphat ((NH₄)2HPO₄); die 10% (NH₄)2HPO₄ sind folglich dem Zementpulver zuzurechnen, woraus sich eine Gesamtpulvermenge von 84% ergibt. | | | | | | | |

### Als Öl wurde Miglyol 812 verwendet

Die Aufstellung der Zementformulierungen zeigt, dass das Prinzip der Nutzung von wasserfreien und mit Wasser nicht im chemischen Sinn mischbaren Flüssigkeiten als Trägerflüssigkeiten für mineralische hydraulische Knochenzemente sehr vielseitig, universell anwendbar und nutzbringend ist.

### Ausführungsbeispiel 4, weitere Zweikomponenten-Zemente

Zweikomponenten-Zemente gemäß nachfolgender Rezeptur wurden nach Ausführungsbeispiel 1b formuliert und getestet

| Zweipasten-Zemente (2-Komponenten-Zemente) | | | | | | | |
|---|---|---|---|---|---|---|---|
| Typ | Zementpulver | Öl | Tween | Amphisol | Beschleuniger/Pa ste 2 | Druckfestigkeit | Abbindezeit |
| CPC | 80% | 17% | 2% | 1% | 100% Wasser | 14 MPa | 8 min |
| MgPC *) | 75% | 13% | 0,8% | 0,8% | 10% (NH4)2HPO4 in Paste1; Paste 2: 28% (NH4)2HPO4, 3% CMS, 1% Span, 68% Wasser | 23 MPa | 5 min |
| Brushite Zement | 81% | 17% | 1% | 1% | Paste 2: 4% Na2HPO4, 3% CMS, 1% Span, 92% Wasser | 4,5 MPa | 6 min |
| HA-Zement | 81% | 17% | 1% | 1% | Paste 2: 4% Na2HPO4, 3% CMS, 1% Span, 92% Wasser | 6,4 MPa | 12 min |

| | | | | | | | |
|---|---|---|---|---|---|---|---|
| CMS: Carboxymethylstärke Span: Tensid *) die Gesamtmenge an Zementpulver beträgt in diesem Beispiel 85%, da die wasserfreie Paste 10% (NH₄)2HPO₄ als Feststoff enthält und zusätzlich ein Teil dieses Ammoniumsalzes in der wässrigen Anmischlösung gelöst vorliegt | | | | | | | |

Brushite Zement: Zementpulver nach Pittet C, Lemaitre J. Mechanical characterization of brushite cements: A Mohr circles' approach, J Biomed Mater Res 2000; 53 (6) 769-780

HA-Zement: Zementpulver nach Brown WE, ChowLC. A new calcium phosphate water setting cement. In: Brown PW, editor. Cements research progress. Westerville, OH: American Ceramic Society; 1986. p. 352-79.

Alle Feststoff enthaltenden Pasten (jeweils Paste 1) waren durch entsprechend eingestellte Mengenverhältnisse von Pulver und Öl auf eine Konsistenz von Erdnussbutter bei Raumtemperatur eingestellt.

Die dargestellten Ergebnisse sind Messwerte aus Machbarkeitsuntersuchungen. Es ist davon auszugehen, dass mit allen untersuchten Zementtypen bei weiteren Optimierungsarbeiten erhebliche Steigerungen in den Festigkeitswerten erreicht werden können. Die getesteten MgCaP-Zemente zeigen bereits nach den ersten Untersuchungen sehr gute mechanische Eigenschaften. Die Abbindezeiten liegen ebenfalls bereits in den vorliegenden Untersuchungen im Bereich der bekannten, entsprechenden Pulver-Flüssigkeits-Varianten.

### Abkürzungen

- ACP: amorphes Calciumphosphat
- CdHA: Calcium-defizientem Hydroxylapatit
- CP: Calciumphosphat
- CPC: Calciumphosphatzement
- DCPA: Dicalciumphosphat Anhydrit
- DCPD: Dicalciumphosphat Dihydrat
- HA: Hydroxylapatit
- Miglyol 812: gesättigtes Triglycerid mit Fettsäuren der Kettenlängen von 8-12
- MCPA: Monocalciumphosphat Anhydrit
- MCPM: Monocalciumphosphat Monohydrat
- OCP: Octacalciumphosphat
- SDS: Sodiumdodecylsulphat
- TCP: Tricalciumphosphat
- TTCP: Tetracalciumphosphat

## Patentansprüche

1. Paste, Suspension oder Dispersion zur Herstellung eines Implantatmaterials auf Basis eines hydraulischen Zements umfassend mindestens eine reaktive mineralische oder organomineralische Zementkomponente die bei Einbringung in oder Vermischung mit Wasser oder einer eine wässrige Lösung enthaltenden Komponente in einer zementartigen Abbindereaktion reagiert und einen Festkörper bildet, **dadurch gekennzeichnet, dass** mindestens ein Calcium- und/oder Magnesiumverbindungen-haltiger pulverförmiger und mit Wasser reaktiver Feststoff mit einer organischen Trägerflüssigkeit, die nicht oder nur < 25 % bezogen auf das Volumen in Wasser löslich ist und in der Wasser nicht oder nur < 25 % bezogen auf das Volumen löslich ist und die bei Vermischung mit Wasser eine Emulsion bildet als Paste, Suspension oder Dispersion formuliert ist, die bei 25 °C und 101,3 kPa eine flüssige bis pastöse Konsistenz aufweist, wobei die Paste, Suspension oder Dispersion ein oder mehrere Tenside enthält und die Paste, Suspension oder Dispersion wasserfrei ist.

2. Paste, Suspension oder Dispersion nach Anspruch 1, **dadurch gekennzeichnet, dass** der Feststoffgehalt in der Paste, Suspension oder Dispersion bei > 2:1 (Masse des Pulvers : Masse der Trägerflüssigkeit) liegt.

3. Paste, Suspension oder Dispersion nach Anspruch 1 oder 2, **dadurch gekennzeichnet, dass** die dispergierten Feststoffe zu > 70 Gew.-% in der aus Flüssigkeit und Feststoffen bestehenden Paste, Suspension oder Dispersion enthalten sind.

4. Paste, Suspension oder Dispersion nach einem der Ansprüche 1 bis 3, **dadurch gekennzeichnet, dass** die Paste, Suspension oder Dispersion gelöste oder suspendierte Polymere mit einem Molekulargewicht von > 2500 enthält.

5. Mehrkomponenten Applikationssystem mit einer Mischvorrichtung, enthaltend eine Paste, Suspension oder Dispersion nach einem der Ansprüche 1 bis 4.

6. Mehrkomponenten Applikationssystem nach Anspruch 5, wobei als weitere Komponente eine wässrige Lösung enthalten ist, wobei die Paste, Suspension oder Dispersion und die wässrige Lösung in getrennten Kartuschen abgefüllt sind.

7. Verwendung einer Paste, Suspension oder Dispersion nach einem der Ansprüche 1 bis 4 zur Herstellung von Knochenzementen, Knochenersatzmaterialien, Knochenklebern, Zahnfüllmaterialien und/oder implantierbaren Wirkstoffträgern.

8. Verfahren zur Herstellung von Knochenzementen, Knochenersatzmaterialien, Knochenklebern, Zahnfüllmaterialien und/oder implantierbaren Wirkstoffträgern, **gekennzeichnet durch** die folgenden Schritte:
- Bereitstellung einer Paste, Suspension oder Dispersion nach einem der Ansprüche 1 bis 4,
- Kombination der Paste, Suspension oder Dispersion mit Wasser oder einer wässrigen Lösung,
wodurch die Paste, Suspension oder Dispersion zu einem Feststoff abbindet.

9. Paste, Suspension oder Dispersion nach einem der Ansprüche 1 bis 4 in Kombination mit Applikationssystemen zur Anwendung bei der Augmentierung von osteoporotischen oder sonstigen pathologisch veränderten Knochenbereichen und zur Auffüllung von Knochendefekten aller Art.

## Claims

1. Paste, suspension or dispersion for the manufacturing of an implant material on the basis of a hydraulic cement comprising at least one reactive mineral or organo-mineral cement component that, after introduction into an aqueous liquid or upon mixing with an aqueous liquid reacts in a cement-like curing reaction and cures to a solid body, **characterized in that** at least one powdery solid, containing calcium and/or magnesium compounds and being reactive with water is formulated together with an organic carrier liquid, which is not soluble in water or only soluble in water to less than 25% in relation to the volume and in which water is not soluble or only soluble to less than 25% in relation to the volume and which forms an emulsion if mixed with water, as paste, suspension or dispersion, which features a liquid up to paste-like consistency at 25 °C and 101.3 kPa, whereas the paste, suspension or dispersion contains one or more tensides and whereas the paste, suspension or dispersion is free from water.

2. Paste, suspension or dispersion according to claim 1, **characterized in that** the solid content in the paste, suspension or dispersion is > 2:1 (mass of the powder : mass of the carrier liquid).

3. Paste, suspension or dispersion according to claim 1 or 2, **characterized in that** the dispersed solids are contained to > 70 % by weight in the paste, suspension or dispersion comprised of liquid and solids.

4. Paste, suspension or dispersion according to claim 1 to 3, **characterized in that** the paste, suspension or dispersion contains dissolved or suspended polymers with a molecular weight of > 2500.

5. Multi-component application system with a mixing device containing a paste, suspension or dispersion according to one of the claims 1 to 4.

6. Multi-component application system according to claim 5, whereat an aqueous solution is comprised, whereat the paste, suspension or dispersion and the aqueous solution are bottled in separated cartouches.

7. Use of a paste, suspension or dispersion according to one of the claims 1 to 4 for producing bone cements, bone replacement materials, bone adhesives, tooth filling materials and/or implantable active ingredient carriers.

8. Method for the production of bone cements, bone replacement materials, bone adhesives, tooth filling materials and/or implantable active ingredient carriers, **characterized by** the following steps:
• providing a paste, suspension or dispersion according to one of the claims 1 to 4,
• combining the paste, suspension or dispersion with water or an aqueous solution,
whereby the paste, suspension or dispersion cures to a solid body.

9. Paste, suspension or dispersion according to one of the claims 1 to 4 in combination with application systems for the application in augmenting osteoporotic or other pathologically modified bone areas and for filling bone defects of all kinds.

## Revendications

1. Pâte, suspension ou dispersion destinée à la préparation d'un matériau d'implant à partir d'un ciment hydraulique comprenant au moins un composant de ciment minéral ou organominéral réactif, qui par ajout à, ou par mélange avec, de l'eau ou un composant contenant une solution aqueuse réagit selon une réaction de durcissement propre au ciment et durcit pour former un solide, **caractérisée par le fait qu'**au moins une matière solide sous forme de poudre contenant un composé de calcium et/ou de magnésium et réagissant avec de l'eau avec un liquide de support organique, qui n'est pas soluble pas dans de l'eau ou qui est uniquement soluble dans de l'eau selon une proportion de moins de 25 % par rapport au volume et dans l'eau n'est pas soluble ou qui est uniquement soluble selon une proportion de moins de 25 % par rapport au volume et par mélange avec de l'eau forme une émulsion, est formulée en tant que pâte, suspension ou dispersion, qui présente une consistance liquide à pâteuse à 25 °C et à 101,3 kPa, grâce à quoi la pâte, la suspension ou la dispersion contient un ou plusieurs agents tensioactifs et grâce à quoi la pâte, la suspension ou la dispersion est anhydre.

2. Pâte, suspension ou dispersion selon la revendication 1, caractérisée en ce la teneur en matière solide dans la pâte, suspension ou dispersion est supérieure à 2:1 (masse de la poudre:masse du liquide de support).

3. Pâte, suspension ou dispersion selon la revendication 1 ou 2, **caractérisée en ce que** les matières solides dispersées à plus de 70 % en poids dans la pâte, la suspension ou la dispersion composée de liquide et de matière solide, sont incluses.

4. Pâte, suspension ou dispersion selon les revendications 1 à 3, **caractérisée en ce que** la pâte, la suspension ou la dispersion inclut un polymère dissous ou en suspension présentant une masse moléculaire de plus de 2 500.

5. Système d'application de plusieurs composants doté d'un dispositif de mélange, comprenant la pâte, la suspension ou la dispersion selon l'une des revendications 1 à 4.

6. Système d'application de plusieurs composants selon la revendication 5, dans lequel une solution aqueuse est incluse en tant qu'autre composant, dans lequel la pâte, la suspension ou la dispersion et la solution aqueuse sont placées dans des cartouches distinctes.

7. Utilisation d'une pâte, d'une suspension ou d'une dispersion selon l'une des revendications 1 à 4, pour la préparation de ciments pour les os, de matériaux de sels pour les os, de colles pour les os, de matériaux de remplissage de dents et/ou de supports à principe actif implantables.

8. Procédé de préparation de ciments pour les os, de matériaux de sels pour les os, de colles pour les os, de matériaux de remplissage de dents et/ou de supports à principe actif implantables, **caractérisé par** les étapes suivantes :
- la fourniture d'une pâte, d'une suspension ou d'une dispersion selon l'une des revendications 1 à 4,
- l'association de la pâte, de la suspension ou de la dispersion à de l'eau ou à une solution aqueuse,
grâce à quoi la pâte, la suspension ou la dispersion durcit pour former une matière solide.

9. Pâte, suspension ou dispersion selon l'une des revendications 1 à 4 en association avec des systèmes d'application pour une utilisation en vue d'une amélioration d'une zone dentaire modifiée par l'ostéoporose ou par une autre pathologie et pour un remplissage d'imperfections dentaires de tout type.
